# EUROPEAN PATENT SPECIFICATION

(11) **EP 0 743 558 B1**
(45) Date of publication and mention of the grant of the patent: **03.08.2005**
(21) Application number: 96107430.9
(22) Date of filing: 10.05.1996
(51) Int. Cl.: G03C 7/42, G03C 5/42, G03C 5/44, C07C 229/76

(54) **Photographic processing composition and processing method**
Photographische Zusammensetzung und photographisches Verfahren
Composition et méthode de traitement photographique

(30) Priority: 12.05.1995 JP 13746595
(43) Date of publication of application: 20.11.1996
(73) Proprietor: FUJI PHOTO FILM CO., LTD., Kanagawa-ken (JP)
(72) Inventor: Inaba, Tadashi, Minami Ashigara-shi, Kanagawa (JP); Okada, Hisashi, Minami Ashigara-shi, Kanagawa (JP); Suzuki, Ryo, Minami Ashigara-shi, Kanagawa (JP)
(74) Representative: Grünecker, Kinkeldey, Stockmair & Schwanhäusser Anwaltssozietät

(56) References cited:
- EP-A- 0 520 457
- EP-A- 0 534 086
- EP-A- 0 657 777
- EP-A- 0 695 969

## Description

The present invention relates to a processing composition for a silver halide photographic material containing at least one metal chelate compound, more specifically, it relates to a processing composition containing a bleaching agent for use in the bleaching process after color development. Moreover, the present invention relates to a method for processing a silver halide photographic material comprising processing an imagewise exposed silver halide photographic material with said processing composition.

In general, a silver halide black-and-white photographic material is exposed and then processed through processing steps such as black-and-white development, fixing and water washing, and a silver halide color photographic light-sensitive material (hereinafter referred to as a color photographic material) is exposed and then processed through processing steps such as color development, desilvering and water washing/stabilization. A silver halide color reversal light-sensitive material is subjected to exposure, black-and-white development and reversal processing, and then processed through processing steps such as color development, desilvering, water washing and stabilization.

In developing a color photographic material, sensitized silver halide grains are reduced at the time of color development by a color developing agent and the produced oxidation product of the color developing agent reacts with a coupler to form an image dye.

In the subsequent desilvering step, the developed silver produced at the color development step is oxidized into silver salt (bleaching) by a bleaching agent (oxidizing agent) having an oxidation action and removed together with unused silver halide from the light-sensitive layer by a fixing agent capable of forming a soluble silver (fixing). The bleaching and the fixing may be conducted individually as a bleaching step and a fixing step or may be conducted simultaneously as a bleach-fixing step. These processing steps are described in detail in James, The Theory of Photographic Process, 4th edition (1977), Research Disclosure, No. 17643, pp. 28-29, ibid., No. 18716, p. 651, from left to right columns, and ibid., No. 307105, pp. 880-881.

In addition to the above-described fundamental processing steps, in order to maintain photographic and physical quality of the dye image or in order to keep the processing stability, various auxiliary processing steps are additionally conducted, such as water washing, stabilization, hardening and stopping.

Further, in order to control the gradient of the developed silver halide black-and-white light-sensitive material, processing with a reducer containing an oxidizing agent is carried out.

The above-described processing is usually conducted in an automatic developing machine. The photographic processing is being conducted in various places including a large-scale processing laboratory where a large-size developing machine is installed and in recent years, a photographic shop where a small-size developing machine called a minilab is installed. Also, rapid processing service is being popularized.

However, the ethylenediaminetetraacetato ferrate complex salt conventionally used as a bleaching agent in the bleaching step or bleach-fixing step in the processing of a color photographic material has a fundamental defect such that the oxidation capability is weak. Accordingly, in spite of the fact that improvement such as the use of a bleaching accelerator (e.g., addition of a mercapto compound described in U.S. Patent 1,138,842) is made, rapid bleaching as a goal has not yet been achieved.

As the bleaching agent capable of rapid bleaching, red prussiate of potash, iron chloride and bromate are known, however, these cannot be widely used because of environmental conservation in case of red prussiate of potash, because of inconvenience in handleability such as metal corrosion in case of iron chloride and because of instability of the solution in case of bromate.

Accordingly, a bleaching solution having good handleability, free from a problem in discharging the waste and capable of rapid bleaching has been demanded. The bleaching agent satisfying these requirements includes 1,3-diaminopropanetetraacetato ferrate complex salt and compounds described in JP-A-3-186841 (the term "JP-A" as used herein means an "unexamined published Japanese patent publication") and JP-A-5-88327.

However, the 1,3-diaminopropanetetraacetato ferrate complex salt is bound to a problem in capability as causing bleach fogging accompanying the bleaching. It is proposed to add a buffer solution to the bleaching solution (for example, in JP-A-1-213657), however, the improvement level is not satisfactory. Further, when the processing solution is adjusted to have a lower pH so as to extract the bleaching capability of the compound, there arise problems in continuous processing such as generation of fog on an unexposed area due to the mingling of the bleaching solution into the color developer, occurrence of desilverization failure due to decomposition of ammonium thiosulfate as the fixing agent caused by the mingling of the bleaching solution into the subsequent fixing solution, and stains on the light-sensitive material surface or clogging of the filter due to formation of precipitate.

Further, the compounds described in JP-A-3-186841 and JP-A-5-88327 can realize rapid bleaching faster than that achieved by the ethylenediaminetetraacetato ferrate complex salt when they are used at a certainly higher concentration, however, when it is used after dilution in view of environmental conservation, the compounds cannot exhibit satisfactory bleaching capability.

Accordingly, a first object of the present invention is to provide a processing composition containing a metal chelate compound less affectable on the photographic properties (e.g., sensitivity, fogging).

A second object of the present invention is to provide a processing composition, particularly, a processing composition having bleaching ability, for a silver halide photographic material, which exhibits excellent desilvering property, and a processing method using the same.

A third object of the present invention is to provide a processing composition, particularly, a processing solution having bleaching ability, for a silver halide color photographic material, which exhibits excellent desilvering property at a diluted concentration, and a processing method using the same.

A fourth object of the present invention is to provide a processing composition, particularly, a processing composition having bleaching ability, for a silver halide color photographic material, which is free of a problem such as generation of fog on an unexposed area even when the bleaching solution is mingled into the color developer in the continuous processing, and a processing method using the same.

A fifth object of the present invention is to provide a processing solution, particularly, a processing solution having bleaching ability, for a silver halide photographic material, which is free from problems such as occurrence of desilverization failure due to decomposition of ammonium thiosulfate as the fixing agent even when the bleaching solution is mingled into the fixing solution in the continuous processing, and stains on the light-sensitive material surface or clogging of the filter due to formation of precipitate.

The present invention provides a processing composition for a silver halide photographic material containing at least one metal chelate compound which acts as an oxidising agent for a silver halide photographic material the chelate compound being represented by the formula (I) 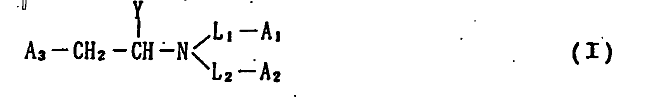 wherein Y represents an optionally substituted cyclic aliphatic hydrocarbon group, a 3- to 10-membered unsaturated or saturated heterocyclic group having at least one of N, O and S atoms which may be monocyclic or may be condensed with another ring to form a condensed ring, or an optionally substituted aryl group; L₁ and L₂ each represents an optionally substituted alkylene group; and A₁, A₂ and A₃ each represents a carboxyl group, a phosphono group, a sulfo group or a hydroxy group,
provided that the processing composition is not a processing solution having a silver bleaching ability comprising a ferric complex salt of 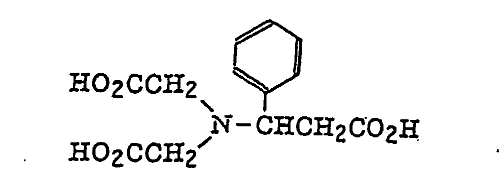 and either a persulfate or a compound of formula 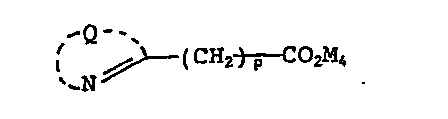 wherein Q represents a nonmetallic atomic group necessary for forming a nitrogen-containing heterocyclic ring; p represents 0 or 1; and M₄ represents a hydrogen atom or a cation.

The present invention further provides a method for processing a silver halide photographic material comprising processing an imagewise exposed silver halide photographic material with the processing composition as defined above.

Preferred embodiments of the present invention are set forth in the sub-claims.

The compound represented by formula (I) (hereinafter referred to as the compound used in the present invention) is described in detail below.

Y represents a cyclic aliphatic hydrocarbon group, a heterocyclic group or an aryl group. The cyclic aliphatic hydrocarbon group represented by Y may be monocyclic or bicyclic. Also, the cyclic aliphatic hydrocarbon group represented by Y may be a saturated aliphatic hydrocarbon group or an unsaturated aliphatic hydrocarbon group, excluding an aryl group. The number of carbon atoms constituting the cyclic aliphatic hydrocarbon group is from 3 to 20, preferably 5 or 6, more preferably 6. Examples of the cyclic aliphatic hydrocarbon group include a cyclohexyl group, a cyclopentyl group, a cyclohexenyl group and a cyclohexadienyl group. When the cyclic aliphatic hydrocarbon group has an unsaturated bond, the number of unsaturated bonds is preferably from 1 to 5, more preferably 1 or 2. The unsaturated bond may be positioned at any site of the cyclic aliphatic hydrocarbon group. The cyclic aliphatic hydrocarbon group represented by Y is preferably a monocyclic ring, more preferably an unsaturated monocyclic ring, most preferably a cyclohexyl group. The cyclic aliphatic hydrocarbon group may have a substituent and examples of the substituent include an alkyl group (preferably having from 1 to 10, more preferably from 1 to 5 carbon atoms, e.g., methyl, ethyl, iso-propyl), an aralkyl group (preferably having from 6 to 20, more preferably from 6 to 10 carbon atoms, e.g., phenylmethyl), an alkenyl group (preferably having from 2 to 10, more preferably from 2 to 5 carbon atoms, e.g., allyl), an alkoxy group (preferably having from 1 to 10, more preferably from 1 to 5 carbon atoms, e.g., methoxy, ethoxy), an aryl group (preferably having from 6 to 20, more preferably from 6 to 10 carbon atoms, e.g., phenyl, p-methylphenyl), an acylamino group (preferably having from 2 to 10, more preferably from 2 to 5 carbon atoms, e.g., acetylamino), a sulfonylamino group (preferably having from 1 to 10, more preferably from 1 to 5 carbon atoms, e.g., methanesulfonylamino), a ureido group (preferably having from 1 to 10, more preferably from 1 to 5 carbon atoms, e.g., methylureido), an alkoxycarbonylamino group (preferably having from 2 to 10, more preferably from 2 to 5 carbon atoms, e.g., methoxycarbonylamino), an aryloxycarbonylamino group (preferably having from 7 to 20, more preferably 7 to 10 carbon atoms, e.g., phenyloxycarbonylamino), an aryloxy group (preferably having from 6 to 20, more preferably 6 to 10 carbon atoms, e.g., phenyloxy), a sulfamoyl group (preferably having from 0 to 10, more preferably from 0 to 5 carbon atoms, e.g., methylsulfamoyl), a carbamoyl group (preferably having from 1 to 10, more preferably from 1 to 5 carbon atoms, e.g., carbamoyl, methylcarbamoyl), a mercapto group, an alkylthio group (preferably having from 1 to 10, more preferably from 1 to 5 carbon atoms, e.g., methylthio, carboxymethylthio), an arylthio group (having from 6 to 20, more preferably from 6 to 10 carbon atoms, e.g., phenylthio), a sulfonyl group (preferably having from 1 to 10, more preferably from 1 to 5 carbon atoms, e.g., methanesulfonyl), a sulfinyl group (preferably having from 1 to 10, more preferably from 1 to 5 carbon atoms, e.g., methanesulfinyl), a hydroxy group, a halogen atom (e.g., chlorine, bromine, fluorine), a cyano group, a sulfo group, a carboxyl group, a phosphono group, an amino group (preferably having from 0 to 10, more preferably from 0 to 5 carbon atoms, e.g., methylamino), an aryloxycarbonyl group (preferably having from 7 to 20, more preferably from 7 to 12 carbon atoms, e.g., phenyloxycarbonyl), an acyl group (preferably having from 2 to 20, more preferably from 2 to 10 carbon atoms, e.g., acetyl, benzoyl), an alkoxycarbonyl group (preferably having from 2 to 12, more preferably from 2 to 6 carbon atoms, e.g., methoxycarbonyl), an acyloxy group (preferably having from 2 to 12, more preferably from 2 to 6 carbon atoms, e.g., acetoxy), a nitro group, a hydroxamic acid group and a heterocyclic group (e.g., pyridyl, furyl, thienyl). These substituents each may further be substituted. Among these substituents, preferred are an alkyl group, an alkoxy group, a hydroxy group, a halogen atom, a sulfo group, a carboxyl group, a phosphono group, an acyl group and an amino group, more preferred are an alkyl group, an alkoxy group, a halogen atom, a hydroxy group, a sulfo group and a carboxyl group.

The heterocyclic group represented by Y is a 3-, 4-, 5-, 6-, 7-, 8-, 9- or 10-membered, unsaturated or saturated heterocyclic group having at least one of N, O and S atoms, and the group may be monocyclic or may be condensed with another ring to form a condensed ring. The heterocyclic group is preferably a 5- or 6-membered aromatic heterocyclic group. Specific examples of the heterocyclic group include a pyrrolidyl group, a piperidino group, a piperidyl group, a piperazyl group, a morpholino group, a morpholinyl group, a thienyl group, a furyl group, a pyrrolyl group, an imidazolyl group, a pyrazolyl group, a pyridyl group, a pyrazyl group, a pyridazyl group, a triazolyl group, a triazyl group, an indolyl group, an indazolyl group, a purinyl group, a thiadiazolyl group, an oxadiazolyl group, a quinolyl group, a phthalazyl group, a naphthyridyl group, a quinoxalyl group, a quinazolyl group, a cinnolyl group, a pteridyl group, an acridyl group, a phenanthrolyl group, a phenazyl group, a tetrazolyl group, a thiazolyl group and an oxazolyl group. Among these heterocyclic groups, preferred are a pyrrolyl group, an imidazolyl group, a pyrazolyl group, a pyridyl group, a pyrazyl group, a pyridazyl group, a triazolyl group, a triazyl group, an indolyl group, an indazolyl group, a thiadiazolyl group, an oxadiazolyl group, a quinolyl group, a phthalazyl group, a quinoxalyl group, a quinazolyl group, a cinnolyl group, a tetrazolyl group, a thiazolyl group and an oxazolyl group, more preferred are an imdazolyl group, a pyrazolyl group, a pyridyl group, a pyrazyl group, an indolyl group, an indazolyl group, a thiadiazolyl group, an oxadiazolyl group, a quinolyl group, a thiazolyl group and an oxazolyl group, still more preferred are an imidazolyl group, a pyridyl group and a quinolyl group, and particularly preferred are an imidazolyl group and a pyridyl group.

The aryl group represented by Y may be monocyclic or may be condensed with another ring to form a condensed ring and the aryl group is preferably an aryl group having from 6 to 20, more preferably from 6 to 16, still more preferably from 6 to 12 carbon atoms. The aryl group represented by Y is preferably a monocyclic or bicyclic aryl group and examples thereof include phenyl and naphthyl, with the phenyl group being more preferred. The aryl group represented by Y may have a substituent and examples of the substituent include those described above as the substituent of the cyclic aliphatic hydrocarbon group represented by Y.

Y is preferably a heterocyclic group or an aryl group, more preferably an aryl group.

L₁ and L₂ each represents an alkylene group. The alkylene group represented by L₁ or L₂ is a linear, branched or cyclic alkylene group, preferably a linear alkylene group. The number of carbon atoms constituting the alkylene group is preferably from 1 to 10, more preferably from 1 to 6, still more preferably from 1 to 4. L₁ and L₂ each is preferably methylene, ethylene, trimethylene, methylmethylene, ethylmethylene, 1-methylethylene, 2-methylethylene, 1-ethylethylene or 2-ethylethylene, more preferably methylene, ethylene or methylmethylene, still more preferably methylene. The alkylene group represented by L₁ or L₂ may have a substituent and examples of the substituent include those described above as the substituent of the cyclic aliphatic group represented by Y. The alkylene group represented by L₁ or L₂, however, preferably has no substituent.

A₁, A₂ and A₃ each represents -COOM, -OH, -SO₃M or -PO(OM)₂, wherein M represents a hydrogen atom or a cation. Among the groups represented by A₁, A₂ and A₃, preferred are -COOM or -OH, more preferred is -COOM. The cation represented by M may be an organic cation or an inorganic cation. When 2 or more cations are present within one molecule, the cations may be the same or different. Examples of the cation include ammonium (e.g., ammonium, tetraethylammonium), alkali metal (e.g., lithium, sodium, potassium), alkaline earth metal (e.g., calcium, magnesium, barium) and pyridinium. Among these, preferred are inorganic cations, more preferred are alkali metal and ammonium.

The compound represented by formula (I) is preferably a compound represented by the following formula (III), more preferably a compound represented by the following formula (IV), still more preferably a compound represented by the following formula (V), and most preferably a compound represented by the following formula (VI): 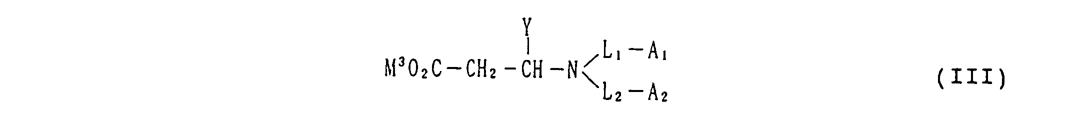 (wherein Y, L₁, L₂, A₁ and A₂ have the same meaning and preferred range as those in formula (I), and M³ has the same meaning and preferred range as the hydrogen atom or the cation represented by M in formula (I)); 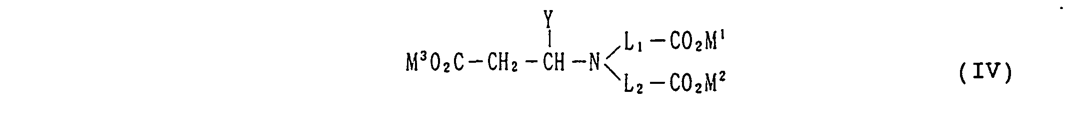 (wherein Y, L₁ and L₂ have the same meaning and preferred range as those in formula (I), and M¹, M² and M³ have the same meaning and preferred range as the hydrogen atom or the cation represented by M in formula (I)); 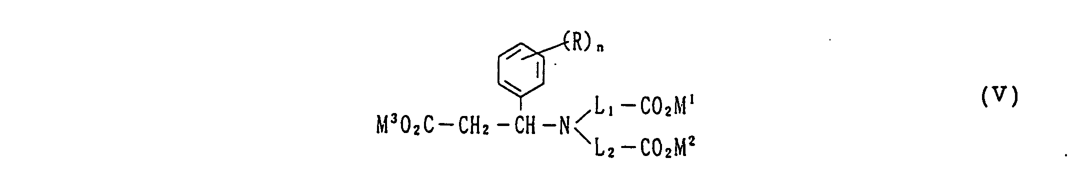 (wherein L₁ and L₂ have the same meaning and preferred range as those in formula (I), M¹, M² and M³ have the same meaning and preferred range as the hydrogen atom or the cation represented by M in formula (I), R represents a substituent and n represents an integer of from 0 to 5).

The substituent represented by R includes those described above as the substituent which the cyclic aliphatic hydrocarbon group represented by Y may have. The substituent is preferably an alkyl group, an alkoxy group, a hydroxy group, a halogen atom, a sulfo group, a carboxyl group, a phosphono group, an acyl group or an amino group, more preferably an alkyl group, an alkoxy group, a halogen atom, a hydroxy group, a sulfo group or a carboxyl group. The substituent may be further substituted with a substituent.

n represents an integer of from 0 to 5, and n is preferably from 0 to 3, more preferably 0 or 1. 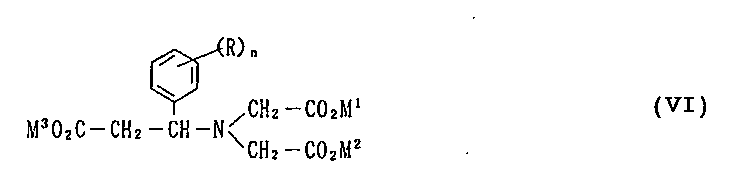 (wherein M¹, M² and M³ have the same meaning and preferred range as the hydrogen atom or the cation represented by M in formula (I) and n represents an integer of from 0 to 5).

Specific examples of the compound represented by formula (I) are described below.

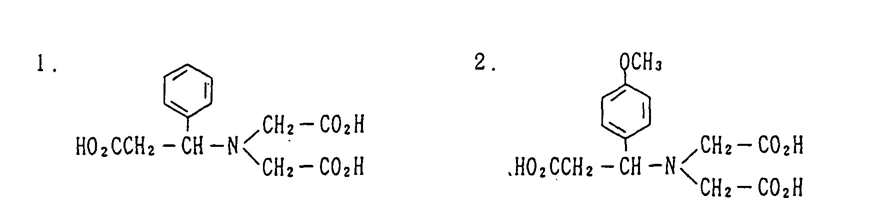

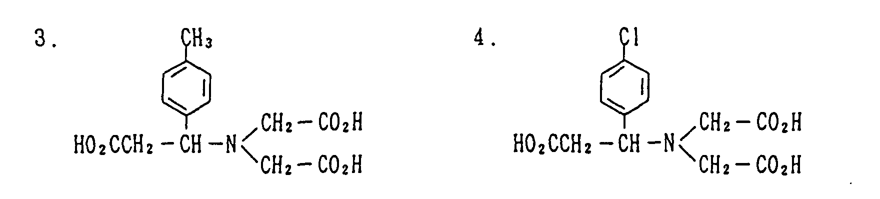

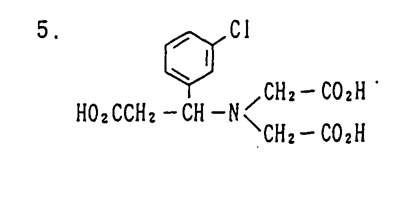

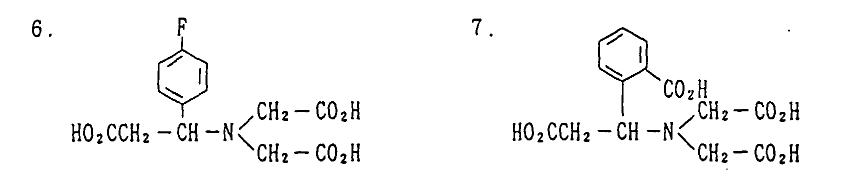

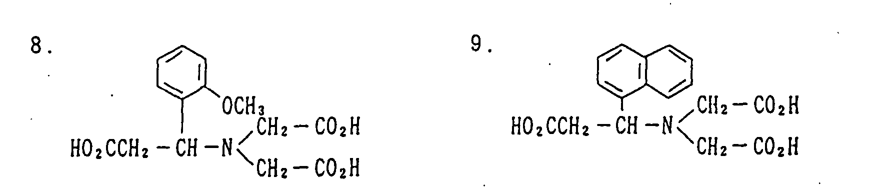

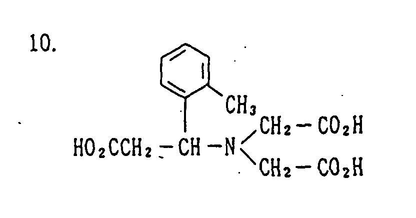

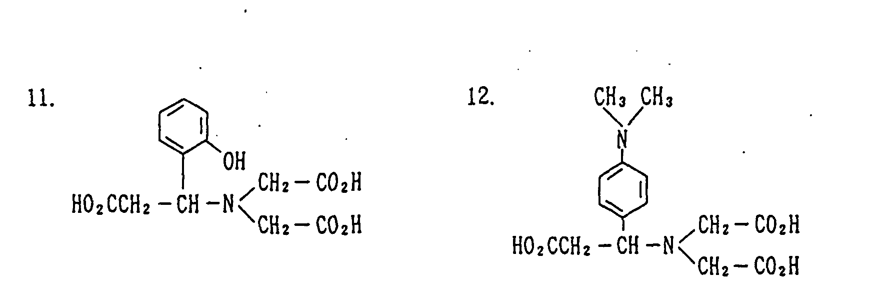

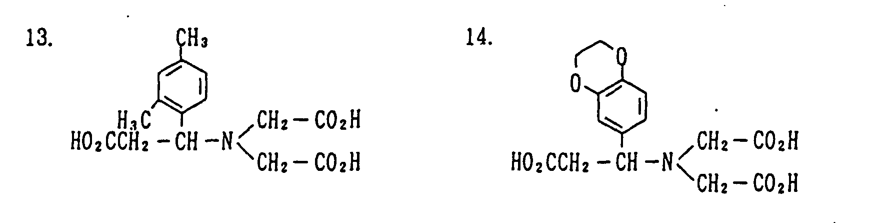

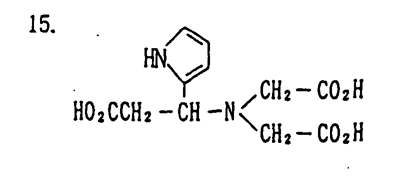

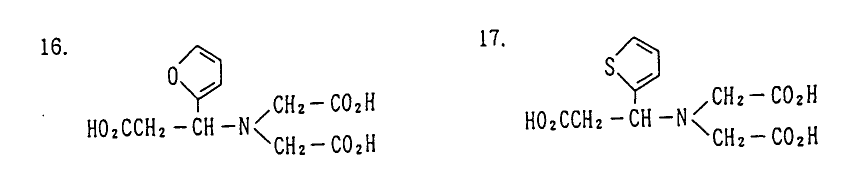

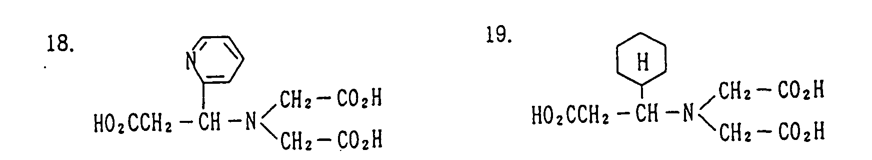

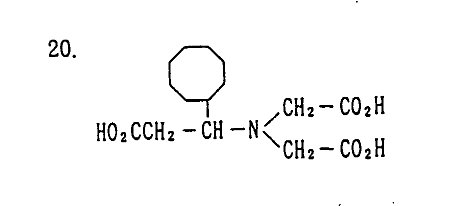

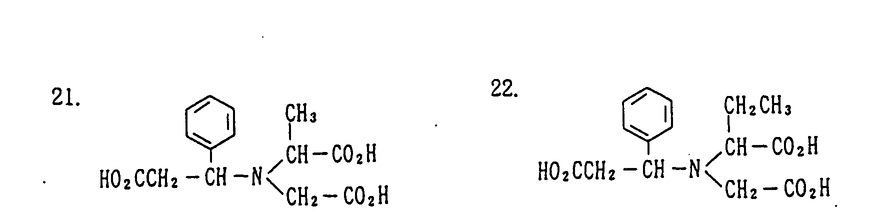

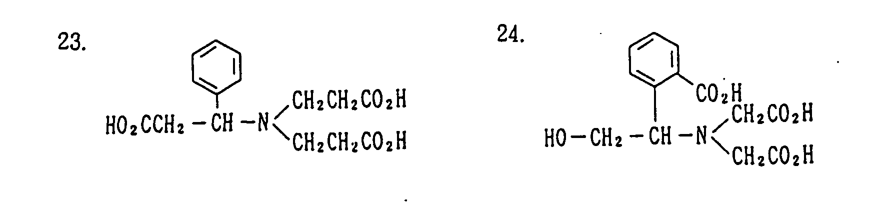

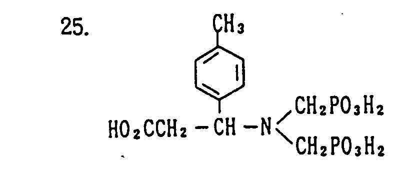

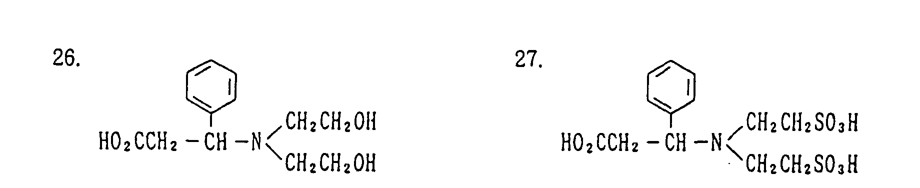

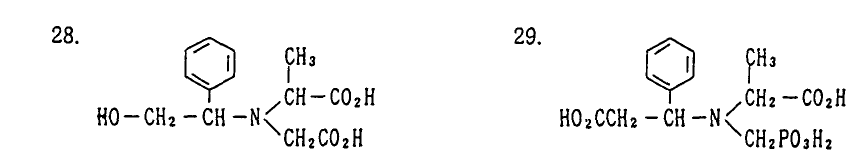

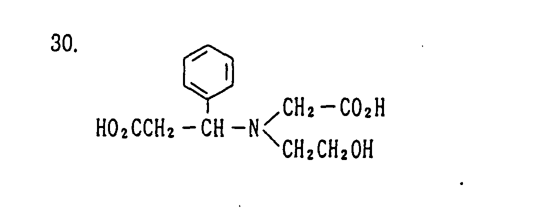

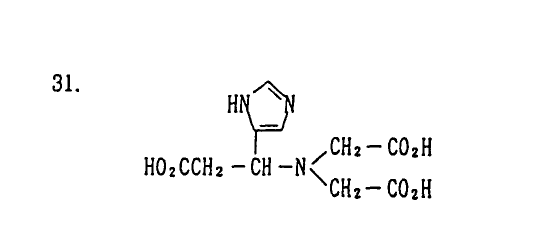

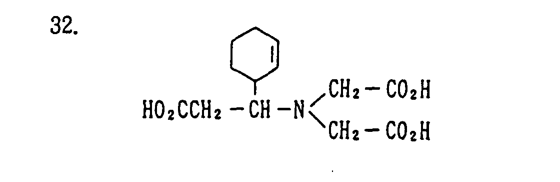

The above-described compounds each may be used in the form of a salt.

The compound represented by formula (I) can be synthesized by the synthesis method of an aminodiacetic acid derivative described in Sverdlovsk, 39-52 (1958) or by a method in accordance thereto. A synthesis method of the compound represented by formula (I) is described below. 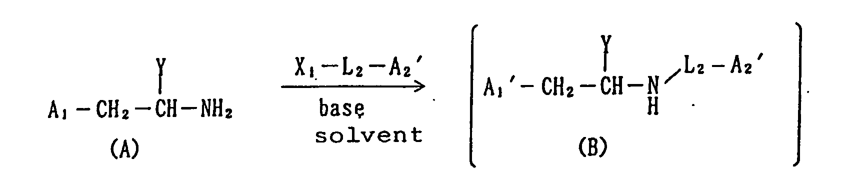 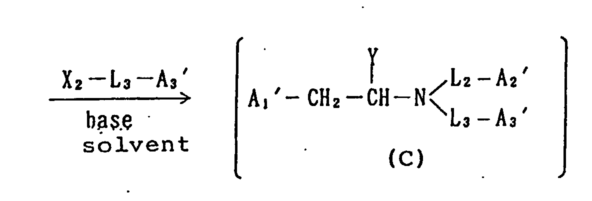 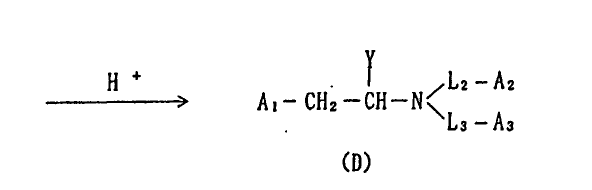 (wherein Y, L₁, L₂, A₁, A₂ and A₃ have the same meaning as those in formula (I), A₁', A₂' and A₃' each represents a carboxyl group, a phosphono group, a sulfo group, a hydroxy group or a salt thereof, and X₁ and X₂ each represents a splitting-off group (for example, a halogen atom (e.g., chlorine, bromine, iodine) or a sulfonate group (e.g., methylsulfonate, p-toluenesulfonate)).

In the scheme above, the amino acid derivative (A) as the starting material can be synthesized from a halogen-substituted alkyl derivative (E) according to the amino group synthesis method described in Jikken Kagaku Koza, 4th ed., vol. 20, pp. 284-288: 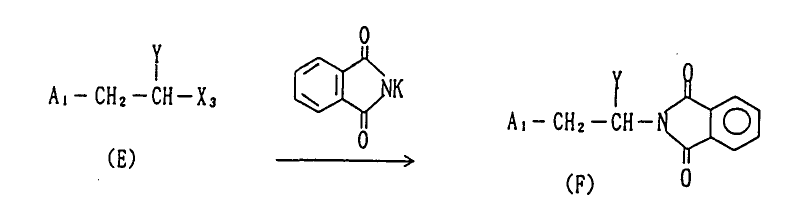 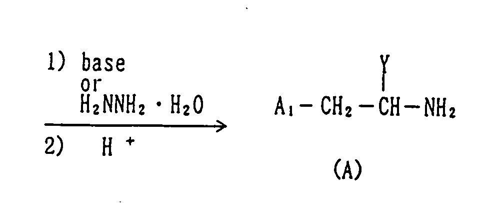 (wherein A₁ and Y have the same meaning as those in formula (I) and X₃ represents a splitting-off group (for example, a halogen atom (e.g., chlorine, bromine, iodine) or a sulfonate group (e.g., methylsulfonate, p-toluenesulfonate)).

When the amino acid derivative (A) is a carboxyl group, the compound can be synthesized by the synthesis method of β-amino acids described in Journal of the American Chemical Society, Vol. 58, 299-303 (1936) or by a method in accordance thereto: 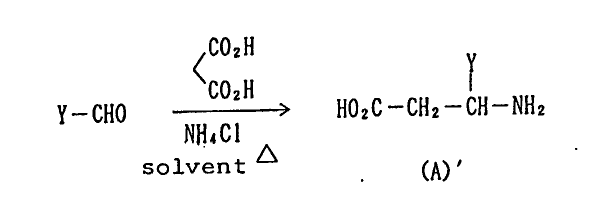 (wherein Y has the same meaning as that in formula (I)).

When X₃ in the halogen-substituted alkyl derivative (E) is a halogen atom, a commercially available compound may be used.

The reaction of the compound of formula (A) or (B) with a halogen-substituted alkylcarboxylic acid may be conducted by referring, in addition to the above-described literature, for example, to Kagehira Ueno, Chelate Kagaku (5), pp. 302-304, Nan'kodo. The reaction of the compound of formula (B) or (C) with a halogen-substituted alkylcarboxylic acid is usually conducted in a solvent at from 0 to 100°C, preferably 70°C or lower. The amount ratio of the halogen-substituted alkylcarboxylic acid used to the compound of formula (B) or (C) is preferably from 1 to 5 times mol, more preferably from 1 to 3 times mol, still more preferably from 1 to 2 times mol.

The solvent used here is not restricted as long as it does not participate in the reaction, however, when water or alcohol (e.g., methanol, ethanol, 2-propanol, butanol) is used, the reaction proceeds advantageously. The reaction is preferably conducted in the presence of a base and examples of the base include an alkali (e.g., sodium hydroxide, potassium hydroxide, sodium carbonate, potassium carbonate) and a tertiary amine (e.g., triethylamine). When water is used as the solvent, the reaction condition is such that the pH is preferably from 7 to 12, more preferably from 7 to 11, still more preferably 8 to 11.

The introduction of the carboxymethyl group in formulae (III), (IV) and (V) may be made according to a Strecker method (see, Jikken Kagaku Koza, 4th ed., vol. 22, pp. 193-195) known as the synthesis method of an amino acid.

The solvent used in the synthesis reaction of Compound (F) is not restricted as long as it does not participate in the reaction, however, when dimethylformacetamido, dimethylformamido, alcohol (e.g., methanol, ethanol, 2-propanol, butanol), acetonitrile, dioxane or tetrahydrofuran is used, the reaction proceeds advantageously.

The solvent used in the synthesis reaction of Compound (A) is not restricted as long as it does not participate in the reaction, however, when water, alcohol (e.g., methanol, ethanol, 2-propanol, butanol), acetonitrile, dioxane, tetrahydrofuran, dimethylformacetamido or dimethylformamido is used, the reaction proceeds advantageously.

The solvent used in the synthesis reaction of Compound (A)' is not restricted as long as it does not participate in the reaction, however, when alcohol (e.g., methanol, ethanol, 2-propanol, butanol), acetonitrile, dioxane, tetrahydrofuran or dimethylformamido is used, the reaction proceeds advantageously.

Compounds 1 and 2 used in the present invention are synthesized according to the method described in Sverdlovsk, pp. 39-52 (1958).

The metal salt constituting the metal chelate compound of the compound represented by formula (I) (hereinafter sometimes simply referred to as the metal chelate compound used in the present invention) is Fe(III) (e.g., ferric sulfate, ferric chloride, ferric nitrate, ammonium ferric sulfate, ferric phosphate) and additional examples thereof include Mn(III), Co(III), Rh(II), Rh(III), Au(II), Au(III) and Ce(IV). Among these, preferred are Fe(III) and Co(III), more preferred is Fe(III).

The metal chelate compound used in the present invention may be isolated as a metal chelate compound or the compound represented by formula (I) may be reacted with the above-described metal salt in a solution. Similarly, an ammonium salt or an alkali metal salt (e.g., lithium salt, sodium salt, potassium salt) of the compound represented by formula (I) may be reacted with the above-described metal salt in a solution.

The compound represented by formula (I) is used in an amount, in term of a molar ratio to the metal ion, of 1.0 or more. The ratio is preferably larger when the stability of the metal chelate compound is low, and it is usually from 1 to 30.

The metal chelate compound used in the present invention may be added to a silver halide photographic material or may be used in a processing solution (in the form of solution or solid) for use in the development processing.

The metal chelate compound used in the present invention provides an effect as an oxidizing agent for a silver halide photographic material (in particular, as a bleaching agent for a color photographic material).

According to a preferred embodiment of the processing composition containing the metal chelate compound used in the present invention, when an imagewise exposed silver halide color photographic material is color developed and then processed with a processing solution having bleaching ability containing the metal chelating compound used in the present invention as the bleaching agent, developed silver can be very rapidly bleached. Further, production of precipitate, stains on the light-sensitive material surface and clogging of the filter, which are seen in the conventional bleaching agent capable of rapid bleaching, are reduced in the running processing.

The characteristic feature of the present invention resides in the bleaching agent as an oxidizing agent in a photographic processing composition, particularly in a processing composition having bleaching ability for a color photographic material, and with respect to factors such as other materials, those which can be commonly used can be appropriately selected.

The processing solution containing the metal chelate compound used in the present invention is described below.

The metal chelate compound used in the present invention may be incorporated into any processing solution (for example, a fixing solution or an intermediate bath between color development and desilverization), however, a processing solution containing the metal chelate compound used in the present invention in an amount of from 0.005 to 1 mol per ℓ of the processing solution is above all effective as a reducer for a black-and-white light-sensitive material or as a processing solution having bleaching ability (e.g., bleaching solution, bleach-fixing solution) for a color photographic material.

A preferred embodiment of the processing solution having bleaching ability is described below. As described above, a processing solution having bleaching ability is effective as the bleaching agent when it contains the metal chelate compound used in the present invention in an amount of from 0.005 to 1 mol, preferably from 0.01 to 0.5 mol, more preferably from 0.05 to 0.5 mol, per ℓ of the processing solution. Also, the metal chelate compound used in the present invention can exhibit excellent capability even when it is used at a diluted concentration of from 0.005 to 0.2 mol, preferably from 0.01 to 0.2 mol, more preferably from 0.05 to 0.18 mol, per ℓ of the processing solution.

The metal chelate compound may be added to the processing solution having bleaching ability in the form not only of an oxidant (e.g., chelate compound of Fe(III)) but also of a reductant thereof (e.g., chelate compound of Fe(II)).

In the case when the metal chelate compound used in the present invention is used as the bleaching agent in a solution having bleaching ability, other bleaching agent may be used in combination within the range of providing effects of the present invention (preferably, in an amount of 0.01 mol or less, more preferably 0.005 mol or less, per ℓ of the processing solution). Examples of the bleaching agent which can be used in combination include compounds of a polyvalent metal such as iron(III), peroxides, quinones and nitro compounds. Representative examples of the bleaching agent include organic complex salts of iron(III) such as ethylenediaminetetraacetic acid, diethylenetriaminepentaacetic acid, cyclohexanediaminetetraacetic acid, methyliminodiacetic acid and glycoletherdiaminetetraacetic acid; bleaching agents including 1,3-propylenediaminetetraacetato ferrate complex salt described in JP-A-4-121739, from page 4, right lower column to page 5, left upper column; carbamoyl-base bleaching agents described in JP-A-4-73647; bleaching agents having a heterocyclic ring described in JP-A-4-174432; bleaching agents including N-(2-carboxyphenyl)iminodiacetato ferrate complex salt described in European Patent Unexamined Publication No. 520457; bleaching agents including ethylenediamine-N-2-carboxyphenyl-N,N',N'-triacetato ferrate acetic acid described in European Patent Unexamined Publication No. 530828A1; bleaching agents described in European Patent Unexamined Publication No. 501479; bleaching agents described in European Unexamined Patent Publication No. 567126; bleaching agents described in JP-A-4-127145; and aminopolycarboxylic acid ferrate and salts thereof described in JP-A-3-144446, page (11).

The processing solution having bleaching ability containing the metal chelate compound used in the present invention contains the metal chelate compound as a bleaching agent and in addition, preferably contains a halide such as chloride, bromide or iodide, as a rehalogenating agent for accelerating oxidation of silver. In place of the halide, an organic ligand capable of forming a difficultly soluble silver salt may be added. The halide is added in the form of an alkali metal salt, ammonium salt or a salt of guanidine or amine. Specific examples thereof include sodium bromide, ammonium bromide, potassium chloride, guanidine hydrochloride, potassium bromide and potassium chloride. In the processing solution having bleaching ability of the present invention, the rehalogenating agent is suitably used in an amount of 2 mol/ℓ or less, and in case of the bleaching solution, it is used in an amount of preferably from 0.01 to 2.0 mol/ℓ, more preferably from 0.1 to 1.7 mol/ℓ, still more preferably from 0.1 to 0.6 mol/ℓ. In case of the bleach-fixing solution, the rehalogenating agent is used in an amount of preferably from 0.001 to 2.0 mol/ℓ, more preferably from 0.001 to 1.0 mol/ℓ, still more preferably from 0.001 to 0.5 mol/ℓ.

The bleaching solution or bleach-fixing solution of the present invention may additionally contain a bleaching accelerator, an anticorrosion for preventing corrosion of the processing bath, a buffer agent for maintaining the pH of the solution, a fluorescent brightening agent and a defoaming agent, if desired.

Examples of the bleaching accelerator include compounds having a mercapto group or a disulfide group described in U.S. Patent 3,893,858, German Patent 1,290,821, British patent 1,138,842, JP-A-53-95630 and Research Disclosure No. 17129 (July, 1978); thiazolidine derivatives described in JP-A-50-140129; thiourea derivatives described in U.S. Patent 3,706,561; iodides described in JP-A-58-16235; polyoxyethylene oxides described in German Patent 2,748,430; and polyamine compounds described in JP-B-45-8836 (the term "JP-B" as used herein means an "examined Japanese patent publication"). Further, compounds described in U.S. Patent 4,552,834 are also preferred. The bleaching accelerator may be added into the light-sensitive material. The above-described bleaching accelerator is particularly effective in bleach-fixing a color photographic material for photographing. In particular, mercapto compounds described in British Patent 1,138,842 and JP-A-2-290856 are preferred.

The bleaching solution or the bleach-fixing solution of the present invention has a pH of from 2.0 to 8.0, preferably from 3.0 to 7.5. When a light-sensitive material for photographing is bleached or bleach-fixed immediately after the color development, the solution is preferably used at a pH of 7.0 or less, preferably 6.4 or less so as to suppress the bleach fogging. Particularly, in the case of a bleaching solution, it is preferably used at a pH of from 3.0 to 5.0. If the pH is 2.0 or lower, the metal chelate used in the present invention readily becomes unstable, thus, the pH is preferably from 2.0 to 6.4. In processing a color printing material, the pH is preferably from 3 to 7.

A pH buffer agent used to this effect may be any as long as it is hardly oxidized by the bleaching agent and exhibits the buffering action in the above-described pH range. Examples of the pH buffer agent include organic acids such as acetic acid, glycolic acid, lactic acid, propionic acid, butyric acid, malic acid, chloroacetic acid, levulinic acid, ureidopropionic acid, formic acid, pyruvic acid, isobutyric acid, pivalic acid, aminobutyric acid, valeric acid, isovaleric acid, asparagine, alanine, arginine, ethionine, glycine, glutamine, cysteine, serine, methionine, leucine, histidine, benzoic acid, hydroxybenzoic acid, nicotinic acid, oxalic acid, malonic acid, succinic acid, tartaric acid, maleic acid, fumaric acid, oxalacetic acid, glutaric acid, adipic acid, aspartic acid, glutamic acid, cystine, ascorbic acid, phthalic acid and terephthalic acid; and organic bases such as pyridine, dimethylpyrazole, 2-methyl-o-oxazoline, aminoacetonitrile and imidazole. These buffer agents may be used in combination. In the present invention, organic acids having an acid dissociation constant (pKa) of from 2.0 to 5.5 are preferred, and dibasic acids are more preferred. Particularly preferred examples of the dibasic acid include a succinic acid, a glutaric acid, a maleic acid, a fumaric acid, a malonic acid and an adipic acid. Among these, most preferred are a succinic acid, a glutaric acid and a maleic acid. The above-described organic acid may also be used in the form of an alkali metal salt (e.g., lithium salt, sodium salt, potassium salt) or an ammonium salt. The buffer agent is suitably used in an amount of 3.0 mol or less, preferably from 0.1 to 2.0 mol, more preferably from 0.2 to 1.8 mol, still more preferably from 0.4 to 1.5 mol, per ℓ of the processing solution having bleaching ability.

In order to adjust the pH of the processing solution having bleaching ability to fall in the above-described range, the acid described above may be used in combination with an alkali agent (e.g., aqueous ammonia, KOH, NaOH, potassium carbonate, sodium carbonate, imidazole, monoethanolamine, diethanolamine). Particularly, aqueous ammonia, KOH, NaOH, potassium carbonate and sodium carbonate are preferably used.

As the anticorrosion, a nitrate is preferably used and examples thereof include ammonium nitrate, sodium nitrate and potassium nitrate. The addition amount thereof is from 0.01 to 2.0 mol/ℓ, preferably from 0.05 to 0.5 mol/ℓ.

In recent years, recognition of environmental conservation of the globe increases and to cope with the trend, an effort is being made to reduce the amount of nitrogen atom to be discharged into the ambient. In this viewpoint, the processing solution of the present invention is preferably demanded to contain substantially no ammonium ion.

The term "contain substantially no ammonium ion" as used in the present invention means the state that the ammonium ion concentration is 0.1 mol/ℓ or less, preferably 0.08 mol/ℓ or less, more preferably 0.01 mol/ℓ or less, and particularly preferably, ammonium ion is not contained at all.

In order to reduce the ammonium ion concentration to the region of the present invention, an alternative cation is required and it is preferably an alkali metal ion or an alkaline earth metal ion, more preferably an alkali metal ion. Among these, preferred are lithium ion, sodium ion and potassium ion and specific examples thereof include sodium salt and potassium salt of an organic acid ferric complex as the bleaching agent, potassium bromide and sodium bromide as the rehalogenating agent in the processing solution having bleaching ability, and potassium nitrate and sodium nitrate.

Preferred examples of the alkali agent used for adjusting the pH include potassium hydroxide, sodium hydroxide, potassium carbonate and sodium carbonate.

The processing solution having bleaching ability of the present invention is particularly preferably aerated at the time of processing because the photographic capability can be held very stably. The aeration can be conducted using a technique known in the art and, for example, the blowing of air or the absorption of air using an ejector into the processing solution may be used.

In blowing air, the air is preferably released into the solution through a diffusion tube having micropores. The tube diffuser is widely used, for example, in the aeration tank for processing activated sludge. With respect to the aeration, the matters described in Z-121, Using Process·C-41, 3rd ed., pages BL-1 to BL-2, issued by Eastman Kodak Co. (1982) may be used. In the processing using the processing solution having bleaching ability of the present invention, stirring is preferably intensified and for intensifying the stirring, the description of JP-A-3-33847, page 8, from right upper column, line 6 to left lower column line 2, may be wholly used.

The bleaching or bleach-fixing may be conducted at a temperature of from 30 to 60°C, but it is preferably conducted at from 35 to 50°C.

The bleaching and/or bleach-fixing processing time is, in case of a light-sensitive material for photographing, from 10 seconds to 7 minutes, preferably 10 seconds to 4 minutes, and in case of a light-sensitive material for printing, it is from 5 to 70 seconds, preferably from 5 to 60 seconds, more preferably from 10 to 45 seconds. Under the above-described preferred processing conditions, good results such as rapid processing without any increase in stains can be obtained.

The light-sensitive material processed with the processing solution having bleaching ability is then fixed or bleach-fixed. In the case where the processing solution having bleaching ability is a bleach-fixing solution, the fixing or bleach-fixing subsequent thereto may be omitted. As the fixing solution or bleach-fixing solution, those described in JP-A-3-33847, from page 6, right lower column, line 16 to page 8, left upper column, line 15, are preferably used.

As the fixing agent used in desilverization, ammonium thiosulfate has been commonly used, however, it may be replaced by other known fixing agents such as a meso-ionic compound, a thioether-base compound, a thiourea, a large amount of iodide, or hypo. These are described in JP-A-60-61749, JP-A-60-147735, JP-A-64-21444, JP-A-1-201659, JP-A-1-210951, JP-A-2-44355 and U.S. Patent 4,378,424. Examples thereof include ammonium thiosulfate, sodium thiosulfate, potassium thiosulfate, guanidine thiosulfate, ammonium thiocyanate, sodium thiocyanate, potassium thiocyanate, dihydroxyethyl-thioether, 3,6-dithia-1,8-octanediol and imidazole, Among these, thiosulfate and mesoions are preferred. In view of rapid fixing property, ammonium thiosulfate is preferred, however, as described above, in view of environmental concern, sodium thiosulfate and mesoions are more preferred so that the processing solution contains substantially no ammonium ion. Two or more kinds of fixing agents may be used in combination to achieve further rapid fixing. For example, in addition to ammonium thiosulfate or sodium thiosulfate, the above-described ammonium thiocyanate, imidazole, thiourea or thioether is preferably used in combination. In this case, the second fixing agent is preferably added in an amount of from 0.01 to 100 mol% to the ammonium thiosulfate or sodium thiosulfate.

The amount of the fixing agent is from 0.1 to 3.0 mol, preferably from 0.5 to 2.0 mol, per ℓ of the bleach-fixing or fixing solution. The pH of the fixing solution varies depending upon the kind of fixing agent, however, it is generally from 3.0 to 9.0 and in particular, when thiosulfate is used, it is preferably from 5.8 to 8.0 so as to achieve stable fixing capability.

The bleach-fixing solution or fixing solution may contain a preservative so as to increase the aging stability of the solution. In the case of the bleach-fixing or fixing solution containing thiosulfate, the effective preservative is a sulfite and/or a bisulfite adduct of hydroxylamine, hydrazine or aldehyde (e.g., bisulfite adduct of acetaldehyde, particularly preferably, an aromatic bisulfite adduct described in JP-A-1-298935). Sulfinic acid compounds described in JP-A-62-143048 can also be preferably used.

The bleach-fixing or fixing solution preferably contains a buffer agent so as to maintain the pH of the solution constant. Examples of the buffer agent include phosphate, imidazoles such as imidazole, 1-methylimidazole, 2-methylimidazole and 1-ethylimidazole, triethanolamine, N-allylmorpholine and N-benzoylpiperazine.

The fixing solution may contain various chelating agents so as to mask iron ions carried over from the bleaching solution to thereby improve stability of the solution. Preferred examples of the chelating agent to this effect include 1-hydroxyethylidene-1,1-diphosphonic acid, nitrilotrimethylenephosphonic acid, 2-hydroxy-1,3-diaminopropanetetraacetic acid, ethylenediaminetetraacetic acid, diethylenetriaminepentaacetic acid, ethylenediamine-N-(β-hydroxyethyl)-N,N',N'-triacetic acid, 1,2-diaminopropanetetraacetic acid, 1,3-diaminopropanetetraacetic acid, nitrilotriacetic acid, cyclohexanediaminetetraacetic acid, iminodiacetic acid, dihydroxyethylglycine, ethyl ether diaminetetraacetic acid, glycol ether diaminetetraacetic acid, ethylenediaminetetrapropionic acid, phenylenediaminetetraacetic acid, 1,3-diaminopropanol-N,N,N',N'-tetramethylenephosphonic acid, ethylenediamine-N,N,N',N'-tetramethylenephosphonic acid, 1,3-propylenediamine-N,N,N',N'-tetramethylenephosphonic acid, serine-N,N-diacetic acid, 2-methylserine-N,N-diacetic acid, 2-hydroxymethylserine-N,N-diacetic acid, hydroxyethyliminodiacetic acid, methyliminodiacetic acid, N-(2-acetamido)iminodiacetic acid, nitrilotripropionic acid, ethylenediaminediacetic acid, ethylenediaminedipropionic acid, 1,4-diaminobutanetetraacetic acid, 2-methyl-1,3-diaminopropanetetraacetic acid, 2-dimethyl-1,3-diaminopropanetetraacetic acid, aniline, tartaric acid, hydrazide dibasic acid, N-hydroxyiminodipropionic acid, alkali metal salts of these (e.g., lithium salt, sodium salt, potassium salt) and ammonium salts of these.

The fixing may be conducted at from 30 to 60°C, but is preferably conducted at from 35 to 50°C.

The fixing processing time is, in case of a light-sensitive material for photographing, from 15 seconds to 2 minutes, preferably from 25 seconds to 1 minute and 40 seconds, and in case of a light-sensitive material for printing, it is from 8 to 80 seconds, preferably from 10 to 45 seconds.

The desilvering is usually conducted using a combination of bleaching, bleach-fixing and fixing. Specific examples of the combination include the following:
(1) bleaching - fixing
(2) bleaching - bleach-fixing
(3) bleaching - bleach-fixing - fixing
(4) bleaching - water washing - fixing
(5) bleach-fixing
(6) fixing - bleach-fixing

In the case of a light-sensitive material for photographing, combinations (1), (2), (3), (4) and (5) are preferred. In the present invention, outstanding effects can be exerted in the processing using a bleach-fixing solution as in combinations (2), (3) and (5), and the processing (5) is particularly preferred.

The present invention may be applied to the desilvering processing via a regulating bath, a stopping bath or a water washing bath after color development.

The processing method of the present invention is preferably carried out in an automatic developing machine. The conveyance method in the automatic developing machine is described in JP-A-60-191257, JP-A-60-191258 and JP-A-60-191259. In order to conduct rapid processing, the crossover between processing tanks is preferably made short. An automatic developing machine where the crossover time is reduced to 5 seconds or less is described in JP-A-1-319038.

In conducting continuous processing according to the processing method of the present invention using an automatic developing machine, a replenisher is preferably added in proportion to the amount of the light-sensitive material processed so as to compensate the consumption of the processing solution components accompanying the processing of the light-sensitive material or to suppress the accumulation of undesired components dissolved out from the light-sensitive material in the processing solution. In each processing step, two or more processing baths may be provided and in this case, a countercurrent system where the replenisher is flown from the post-bath to the pre-bath is preferred. In particular, the water washing or the stabilization is preferably in a cascade system consisting of from 2 to 4 stages.

The amount of the replenisher is preferably reduced as long as the compositional change in each processing solution does not cause any inconvenience in the photographic capability or contamination of other solutions.

The amount of the replenisher for color developer is, in case of a color photographic material, from 50 to 3,000 ml, preferably from 50 to 2,200 ml, per m² of the light-sensitive material, and in case of a color printing material, it is from 15 to 500 ml, preferably from 20 to 350 ml, per m² of the light-sensitive material.

The amount of the replenisher for bleaching solution is, in case of a color photographic material, from 10 to 1,000 ml, preferably from 50 to 550 ml, per m² of the light-sensitive material, and in case of a printing material, it is from 15 to 500 ml, preferably from 20 to 300 ml, per m² of the light-sensitive material.

The amount of the replenisher for bleach-fixing solution is, in case of a color photographic material, from 150 to 3,000 ml, preferably from 180 to 1,300 ml, per m² of the light-sensitive material, and in case of a printing material, it is from 20 to 300 ml, preferably 50 to 200 ml, per m² of the light-sensitive material. The bleach-fixing solution may be replenished by one solution or may be replenished separately by a bleaching composition and a fixing composition, or the overflow solutions from the bleaching bath and/or fixing bath may be mixed to serve as the replenisher for bleach-fixing solution.

The amount of the replenisher for fixing solution is, in case of a color photographic material, from 300 to 3,000 ml, preferably from 300 to 1,200 ml, per m² of the light-sensitive material, and in case of a printing material, it is from 20 to 300 ml, preferably from 50 to 200 ml, per m² of the light-sensitive material.

The replenishing amount of washing water or stabilizing solution is from 1 to 50 times, preferably from 2 to 30 times, more preferably from 2 to 15 times, the amount carried over from the pre-bath per the unit area.

The processing solution having bleaching ability of the present invention may be re-used by recovering the overflow solution after use in the processing and adding thereto components to correct the composition. This use method is usually called regeneration and the regeneration is also preferred in the present invention. With respect to the details of the regeneration, matters described in Fuji Film Processing Manual, Fuji Color Negative Film, CN-16 Processing, issued by Fuji Photo Film Co., Ltd., pp. 39-40 (revised in August, 1990) may be used.

The kit for regulating the processing solution having bleaching ability of the present invention may be liquid or powder, however, if ammonium salt is excluded, almost all raw materials are fed as powder and also low in the hygroscopicity, accordingly, a powder kit is easily prepared.

The kit for the above-described regeneration is preferably powder in view of reduction in the amount of waste because no excessive water is needed and direct addition is possible.

For regenerating the processing solution having bleaching ability, in addition to the above-described aeration, the method described in Shashin Kogaku no Kiso - Gin'en Shashin Hen-, Nippon Shashin Gakkai (compiler), Corona Sha (1979) may be used. More specifically, electrolytic regeneration and other regeneration methods of the bleaching solution by bromic acid, chlorous acid, bromine, bromine precursor, persulfate, hydrogen peroxide, hydrogen peroxide using a catalyst, bromous acid and ozone, may be used.

In the regeneration by electrolysis, a cathode and an anode are placed in the same bleaching bath or an anode tank and a cathode tank are provided as separate baths using a diaphragm to regenerate the solution, or the bleaching solution and the developer and/or the fixing solution may be simultaneously regenerated also using a diaphragm.

The fixing solution and the bleach-fixing solution each is regenerated by the electrolytic reduction of silver ions accumulated. In addition, in order to maintain the fixing capability, halogen ions accumulated is preferably removed using an anion exchange resin.

The use amount of washing water may be reduced using ion exchange or ultrafiltration, but ultrafiltration is particularly preferred.

In the present invention, the color photographic material is subjected to color development after imagewise exposure but before desilverization processing. The color developer which can be used in the present invention include those described in JP-A-3-33847, from page 9, left upper column, line 6 to page 11, right lower column, line 6, and those described in JP-A-5-197107.

The color developing agent used in the color development step may be a known aromatic primary amine color developing agent and a p-phenylenediamine-base compound is preferably used. Representative examples thereof include 3-methyl-4-amino-N,N-diethylaniline, 3-methyl-4-amino-N-ethyl-N-β-hydroxyethylaniline, 3-methyl-4-amino-N-ethyl-N-β-methanesulfonamidoethylaniline, 3-methyl-4-amino-N-ethyl-β-methoxyethylaniline, 4-amino-3-methyl-N-methyl-N-(3-hydroxypropyl)aniline, 4-amino-3-methyl-N-ethyl-N-(3-hydroxypropyl)aniline, 4-amino-3-methyl-N-ethyl-N-(2-hydroxypropyl)aniline, 4-amino-3-ethyl-N-ethyl-N-(3-hydroxypropyl)aniline, 4-amino-3-methyl-N-propyl-N-(3-hydroxypropyl)aniline, 4-amino-3-propyl-N-methyl-N-(3-hydroxypropyl)aniline, 4-amino-3-methyl-N-methyl-N-(4-hydroxybutyl)aniline, 4-amino-3-methyl-N-ethyl-N-(4-hydroxybutyl)aniline, 4-amino-3-methyl-N-propyl-N-(4-hydroxybutyl)aniline, 4-amino-3-ethyl-N-ethyl-N-(3-hydroxy-2-methylpropyl)aniline, 4-amino-3-methyl-N,N-bis(4-hydroxybutyl)aniline, 4-amino-3-methyl-N,N-bis(5-hydroxypentyl)-aniline, 4-amino-3-methyl-N-(5-hydroxypentyl)-N-(4-hydroxybutyl)aniline, 4-amino-3-methoxy-N-ethyl-N-(4-hydroxybutyl)-aniline, 4-amino-3-ethoxy-N,N-bis(5-hydroxypentyl)aniline, 4-amino-3-propyl-N-(4-hydroxybutyl)aniline, and a sulfate, a hydrochloride and a p-toluenesulfonate of these. Among these, particularly preferred are 3-methyl-4-amino-N-ethyl-N-β-hydroxyethylaniline, 4-amino-3-methyl-N-ethyl-N-(3-hydroxypropyl)aniline, 4-amino-3-methyl-N-ethyl-N-(4-hydroxybutyl)-aniline, and a sulfate, a p-toluenesulfonate and a sulfate of these. These compounds may be used in combination of two or more depending on the purpose.

Those described in European Unexamined Patent Publication No. 410450 and JP-A-4-11255 are also preferably used.

Salts of a p-phenylenediamine derivative of these with a sulfate, a hydrochloride, a sulfite, a naphthalenedisulfonic acid or a p-toluenesulfonic acid may also be used. The use amount of the aromatic primary amine developing agent is preferably from 0.0002 to 0.2 mol, more preferably from 0.001 to 0.1 mol, per ℓ of the color developer.

In the present invention, the processing temperature in the processing with a color developer is from 20 to 55°C, preferably from 30 to 55°C. The processing time is, in case of a light-sensitive material for photographing, from 20 seconds to 5 minutes, preferably from 30 seconds to 3 minutes and 20 seconds, more preferably from 1 minute to 2 minute and 30 seconds, and in case of a light-sensitive material for printing, it is from 10 seconds to 1 minute and 20 seconds, preferably from 10 to 60 seconds, more preferably from 10 to 40 seconds.

The processing method of the present invention may be used in the color reversal processing. The black-and-white developer used herein is one called a black-and-white first developer usually used in the reversal processing of a color photographic material. Various well-known additives which are added to the black-and-white developer for use in the processing solution of a black-and-white silver halide light-sensitive material, may be incorporated into the black-and-white first developer for a color reversal light-sensitive material.

Representative examples of the additive include a developing agent such as 1-phenyl-3-pyrazolidone, Metol and hydroquinone, a preservative such as sulfite, an accelerating agent comprising an alkali such as sodium hydroxide, sodium carbonate or potassium carbonate, an inorganic or organic inhibitor such as potassium bromide, 2-methylbenzimidazole and methylbenzothiazole, a hard water softening agent such as a polyphosphate, and a development inhibitor comprising a slight amount of iodide or a mercapto compound.

In the present invention, the desilvered light-sensitive material is subjected to water washing and/or stabilization. In the water washing and the stabilization, a stabilizing solution described in U.S. Patent 4,786,583 may be used. The stabilizing solution uses formaldehyde as a stabilizer, however, in view of the safety in working environment, N-methylolazole, hexamethylenetetramine, formaldehyde bisulfate adduct, dimethylolurea and azolylmethylamine derivative are preferred. These are described in JP-A-2-153348, JP-A-5-34889, JP-A-4-313753 and JP-A-5-165178. In particular, a combination use of an azole such as 1,2,4-triazole with an azolylmethylamine such as 1,4-bis(1,2,4-triazol-1-ylmethyl)piperazine or a derivative thereof (described in JP-A-4-359249) is preferred because the image stability can be high and the formaldehyde vapor pressure is low.

The processing composition for use in the present invention may be supplied in any form such as a liquid agent in a concentration of the solution in the use state or in the condensed form, a granulated powder, a powder, a pellet, a tablet, a paste, a jelly or a latex. Examples of the processing agent include a liquid agent housed in a container having a low oxygen permeability described in JP-A-63-17453, vacuum-packaged powder or granulated powder described in JP-A-4-19655 and JP-A-4-230748, granulated powder having incorporated therein a water-soluble polymer described in JP-A-4-221951, a tablet described in JP-A-51-61837 and JP-A-6-102628, and a paste processing agent described in the unexamined published Japanese patent application No. Shou. 57-500485 based on a PCT application, which all are preferably used, however, in view of convenience on use, a liquid previously regulated to have a concentration in the use state is preferred.

The container for housing the processing agent uses polyethylene, polypropylene, polyvinyl chloride, polyethylene terephthalate and nylon individually or as a composite material. These materials are selected according to the level of the oxygen permeability required. For the solution susceptible to oxidation such as color developer, materials having a low oxygen permeability are preferred and more specifically, a composite material of polyethylene terephthalate or polyethylene and nylon is preferred. The material is used for the container to have a thickness of from 500 to 1,500 µm and an oxygen permeability of preferably 20 ml/m²•24 hrs•atm or less.

Examples of the light-sensitive material to which the processing of the present invention can be applied include a color negative film, a color reversal film (substantive type, non-substantive type), a color paper, a color reversal paper, a color negative film for movie, a color positive film for movie, a color negative slide, a color reversal film for television and a direct positive color paper, which are described, for example, in JP-A-3-33847, JP-A-3-293662 and JP-A-4-130432. There is no particular limitation on the support of the light-sensitive material; the coating method; the kind of silver halide used in the silver halide emulsion layer or the surface protective layer (e.g., silver iodobromide, silver iodochlorobromide, silver bromide, silver chlorobromide, silver chloride), the grain form (e.g., cubic, tabular, spherical), the grain size, the coefficient of variation thereof, the crystal structure (e.g., core/shell structure, multi-phase structure, homogeneous phase structure), the processing method (e.g., single jet method, double jet method), a binder (e.g., gelatin), a hardening agent, an antifoggant, a metal doping agent, a silver halide solvent, a tackifying agent, an emulsion precipitating agent, a dimension stabilizer, an adhesion inhibitor, a stabilizer, a color mixing inhibitor, a dye image stabilizer, a stain inhibitor, a chemical sensitizer, a spectral sensitizer, a sensitivity increasing agent, a supersensitizer, a nucleating agent, a coupler (e.g., pivaloylacetanilde-type or benzoylacetanilide-type yellow coupler, 5-pyrazolone-type or pyrazoloazole-type magenta coupler, phenol-type or naphthol-type cyan coupler, DIR coupler, bleaching accelerator releasing coupler, competitive coupler, colored coupler), a coupler dispersion method (e.g., oil-in-water dispersion using a high boiling point solvent), a plasticizer, an antistatic agent, a lubricant, a coating aid, a surface active agent, a brightening agent, a formalin scavenger, a light scattering agent, a matting agent, a light absorbent, an ultraviolet absorbent, a filter dye, an irradiation dye, a development improving agent, a delustering agent, an antiseptic (e.g., 2-phenoxyethanol) and an antimold, which may be used in the present invention, and those described, for example, in Product Licensing, vol. 92, pp. 107-110 (December, 1971), Research Disclosure (hereinafter referred to as RD), No. 17643 (December, 1978), RD, No. 18716 (November, 1979), and RD, No. 307105 (November, 1989) may be referred to.

The processing method of the present invention may be used for any color photographic material, however, the dry thickness, the swelling ratio and the swelling rate of the entire constituent layers of the color photographic material preferred in the present invention are described in JP-A-5-66527, paragraphs "0099" to "0100". The silver halide contained in the photographic emulsion layer of the color photographic material may be any silver halide composition and examples thereof include silver chloride, silver bromide, silver chlorobromide, silver iodobromide, silver iodochloride and silver iodochlorobromide.

In case of a color photographic material for photographing or a color reversal light-sensitive material (e.g., color negative film, reversal film, color reversal paper), silver iodobromide, silver iodochloride and silver iodochlorobromide each containing from 0.1 to 30 mol% of silver iodide are preferred. In particular, silver iodobromide containing from 1 to 25 mol% of silver iodide is preferred. In case of a direct positive color photographic material using an internal latent image-type emulsion which is not previously fogged, silver bromide and silver chlorobromide are preferred and silver chloride is also preferred in conducting rapid processing. In case of a light-sensitive material for paper, silver chloride and silver chlorobromide are preferred and particularly, silver chlorobromide having a silver chloride content of 80 mol% or more, more preferably 95 mol% or more, most preferably 98 mol% or more is preferred.

The color photographic material applied to the processing of the present invention may use various color couplers and specific examples thereof are described in patents cited in the above-described RD, No. 17643, VII-C to G and ibid., No. 307105, VII-C to G, JP-A-62-215272, JP-A-3-33847, JP-A-2-33144, and European Unexamined Patent Publication Nos. 447969A and 482552A.

As the yellow coupler, those described, for example, in U.S. Patents 3,933,501, 4,022,620, 4,326,024, 4,401,752 and 4,248,961, JP-B-58-10739, British Patents 1,425,020 and 1,476,760, U.S. Patents 3,973,968, 4,314,023, 4,511,649 and 5,118,599, European Patents 249,473A and 0,447,969, JP-A-63-23145, JP-A-63-123047, JP-A-1-250944 and JP-A-1-213648 may be used in combination as long as the effect of the present invention is not impaired.

Preferred examples of the yellow coupler include yellow couplers represented by formula (Y) of JP-A-2-139544, from page 18, left upper column to page 22, left lower column; acylacetamido-base yellow couplers characterized in the acyl group described in JP-A-5-2248 and European Unexamined Patent Publication No. 0447969; and yellow couplers represented by formula (Cp-2) described in JP-A-5-27389 and European Unexamined Patent Publication No. 0446863A2.

As the magenta coupler, 5-pyrazolone-base and pyrazoloazole-base compounds are preferred and those described in U.S. Patents 4,310,619 and 4,351,897, European Patent 73636, U.S. Patents 3,061,432 and 3,725,067, Research Disclosure No. 24220 (June, 1984), JP-A-60-33552, Research Disclosure No. 24230 (June, 1984), JP-A-60-43659, JP-A-61-72238, JP-A-60-35730, JP-A-55-118034, JP-A-60-185951, U.S. Patents 4,500,630, 4,540,654 and 4,556,630 and International Unexamined Patent Publication WO88/04795 are more preferred.

Particularly preferred magenta couplers are pyrazoloazole-base magenta couplers represented by formula (I) of JP-A-2-139544, from page 3, right lower column to page 10, right lower column; and 5-pyrazolone magenta couplers represented by formula (M-1) of JP-A-2-139544, from page 17, left lower column to page 21, left upper column. Most preferred are pyrazoloazole-base magenta couplers described above.

The cyan coupler includes phenol and naphthol couplers and those described in U.S. Patents 4,052,212, 4,146,396, 4,228,233, 4,296,200, 2,369,929, 2,801,171, 2,772,162, 2,895,826, 3,772,002, 3,758,308, 4,334,011 and 4,327,173, West German Patent (OLS) No. 3,329,729, European Patents 0121365A and 0249453A, U.S. Patents 3,446,622, 4,333,999, 4,775,616, 4,451,559, 4,427,767, 4,690,889, 4,254,212 and 4,296,199 and JP-A-61-42658 are preferred. Also, pyrazoloazole-base couplers described in JP-A-64-553, JP-A-64-554, JP-A-64-555 and JP-A-64-556, pyrrolotriazole-base couplers described in European Unexamined Patent Publication Nos. 0488248 and 0491197, pyrroloimidazole-base couplers described in European Unexamined Patent Publication No. 0456226A, pyrazolopyrimidine-base couplers described in JP-A-64-46753, imidazole-base couplers described in U.S. Patent 4,818,672 and JP-A-2-33144, cyclic active methylene-type cyan couplers described in JP-A-64-32260 and couplers described in JP-A-1-183658, JP-A-2-262655, JP-A-2-85851 and JP-A-3-48243 may be used.

Typical examples of the polymerized dye forming coupler are described in U.S. Patents 3,451,820, 4,080,211, 4,367,282, 4,409,320 and 4,576,910, British Patent 2,102,137 and European Patent 341188A.

As the coupler which provides a colored dye having an appropriate diffusibility, those described in U.S. Patent 4,366,237, British Patent 2,125,570, European Patent 96570 and West German Patent (OLS) No. 3,234,533 are preferred.

Compounds which release a photographically useful residue on coupling are also preferably used in the present invention. Preferred DIR couplers which release a development inhibitor are described in patents cited in the above-described RD, No. 17643, Item VII-F, JP-A-57-151944, JP-A-57-154234, JP-A-60-184248, JP-A-63-37346, and U.S. Patents 4,248,962 and 4,782,012.

As the coupler which imagewise releases a nucleating agent or a development accelerator at the development, those described in British Patents 2,097,140 and 2,131,188, JP-A-59-157638 and JP-A-59-170840 are preferred.

Other couplers which can be used in the color photographic element of the present invention include competitive couplers described in U.S. Patent 4,130,427, polyequivalent couplers described in U.S. Patents 4,283,472, 4,338,393 and 4,310,618, DIR redox compound-releasing couplers, DIR coupler-releasing couplers, DIR coupler-releasing redox compounds and DIR redox-releasing redox compounds described in JP-A-60-185950 and JP-A-62-24252, couplers which release a dye capable of recovering the color after being released described in European Patent 173302A, couplers which release a bleaching accelerator described in RD, No. 11449, ibid., No. 24241 and JP-A-61-201247, ligand-releasing couplers described in U.S. Patent 4,553,477, couplers which release a leuco dye described in JP-A-63-75747 and couplers which release a fluorescent dye described in U.S. Patent 4,774,181.

Examples of the support which can be suitably used in the present invention include those described in RD, No. 17643, page 28, and ibid., No. 18716, from page 647, right column to page 648, left column.

With respect to the support for use in a color negative film, one having on one surface thereof an electrically conductive layer and a transparent magnetic substance layer described in JP-A-4-62543, one having a magnetic recording layer described in International Unexamined Patent Application WO90/04205, Fig. 1, and one having a striped magnetic recording layer and a transparent magnetic recording layer adjacent to the striped magnetic recording layer described in JP-A-4-124628 are preferably used. On the magnetic recording layer, a protective layer described in JP-A-4-73737 is preferably provided.

The thickness of the support is preferably from 70 to 120 µm and as the material for the support, various plastic films described in JP-A-4-124636, from page 5, right upper column, line 1 to page 6, right upper column, line 5 can be used. Preferred examples thereof include cellulose derivatives (e.g., diacetylacetate, triacetylacetate, propionylacetate, butanoylacetate, acetylpropionylacetate), and polyesters described in JP-B-48-40414 (e.g., polyethylene terephthalate, poly-1,4-cyclohexanedimethylene terephthalate, polyethylene naphthalate). The support of the film used in the present invention is preferably polyester because a higher solution cutting effect can be obtained.

The package (patrone) for housing a color negative film may be any of currently used or known ones, however, those having a shape described in U.S. Patent 4,834,306, Figs. 1 to 3, and those described in U.S. Patent 4,846,418, Figs. 1 to 3 are preferred.

The color negative film for use in the present invention preferably comprises the content described in JP-A-4-125558, from page 14, left upper column, line 1 to page 18, left lower column, line 11.

The light-sensitive material for use in the present invention is preferably one as described below.

A light-sensitive material having a magnetic recording layer is used and the recording layer comprises a binder having dispersed therein magnetic particles (preferably, Co-coated ferromagnetic iron oxide). The magnetic layer is preferably optically transparent and provided over the entire surface of the light-sensitive material. The magnetic particle may be treated with a coupling agent as described in JP-A-6-161032. The binder is preferably a polymer described in JP-A-4-219569. The recording layer may be provided at any position, but it is preferably provided on the side opposite to the emulsion layer (back layer) of the support. Further, it is preferred that a layer containing a sliding agent is provided as an upper layer on the recording layer and the outermost layer on the light-sensitive emulsion layer side of the support contains a matting agent.

The light-sensitive material preferably contains an antistatic agent so that the antistatic property can be imparted even after the development. The antistatic agent is preferably an electrically conductive metal oxide or an ionic polymer. The antistatic agent is preferably used to exhibit an electric resistance under conditions of 25°C and 10% RH, of 10¹² Ω•cm or less.

The light-sensitive material having a magnetic recording layer is described in U.S. Patents 5,336,589, 5,250,404, 5,229,259 and 5,215,874, and EP466130A.

The support for use in the light-sensitive material is preferably a thin polyester support improved in the curling habit. The thickness is preferably from 50 to 105 µm and the material is preferably a polyethylene aromatic dicarboxylate-base polyester (preferably using benzenedicarboxylic acid, naphthalenedicarboxylic acid and ethylene glycol as main raw materials). The glass transition temperature of the support is preferably from 50 to 200°C. Further, the support is subjected to surface treatment, preferably to ultraviolet irradiation treatment, corona discharge treatment, glow discharge treatment or flame treatment. The support is preferably subjected to heat treatment at a temperature of from 40°C to the glass transition temperature of the support for from 0.1 to 1,500 hours, between before or after the application of an undercoat layer and before the coating of an emulsion layer. The support, the light-sensitive material, the development and the cartridge are described in JIII Journal of Technical Disclosure, No. 94-6023, Hatsumei Kyokai (1994).

The present invention can also be used as a reducer for correcting a silver image comprising dots and/or lines obtained by exposing and then developing a silver halide light-sensitive material for plate making.

### EXAMPLES

The present invention is described below in greater detail with reference to Examples.

### EXAMPLE 1

### Preparation of Multi-Layer Color Photographic Material:

Sample 101, a multi-color photographic material, was prepared by coating layers each having the following composition.

### (Composition of Light-Sensitive Layer)

The main materials used in each layer are classified as follows. ExC: cyan coupler ExM: magenta coupler ExY: yellow coupler ExS: sensitizing dye UV: ultraviolet absorbent HBS: high-boiling point organic solvent H: gelatin hardening agent

Numerals corresponding to respective ingredients show coating amounts expressed by the unit g/m² and in case of silver halide, they show coating amounts in terms of silver. With respect to sensitizing dyes, the coating amount is shown by the unit mol per mol of silver halide in the same layer.

### (Sample 101)

| First Layer (antihalation layer) | |
|---|---|
| Black colloidal silver | as silver 0.18 |
| Gelatin | 1.60 |
| ExM-1 | 0.11 |
| ExF-1 | 3.4×10⁻³ |
| ExF-2 (solid disperse dye) | 0.03 |
| ExF-3 (solid disperse dye) | 0.04 |
| HBS-1 | 0.16 |

| Second Layer (interlayer) | |
|---|---|
| ExC-2 | 0.055 |
| UV-1 | 0.011 |
| UV-2 | 0.030 |
| UV-3 | 0.053 |
| HBS-1 | 0.05 |
| HBS-2 | 0.02 |
| Polyethyl acrylate latex | 8.1×10⁻² |
| Gelatin | 1.75 |

| Third Layer (low-sensitivity red-sensitive emulsion layer) | |
|---|---|
| Silver Iodobromide Emulsion A as silver | 0.46 |
| ExS-1 | 5.0×10⁻⁴ |
| ExS-2 | 1.8×10⁻⁵ |
| ExS-3 | 5.0×10⁻⁴ |
| ExC-1 | 0.11 |
| ExC-3 | 0.045 |
| ExC-5 | 0.0050 |
| ExC-7 | 0.001 |
| ExC-8 | 0.010 |
| Cpd-2 | 0.005 |
| HBS-1 | 0.090 |
| Gelatin | 0.87 |

| Fourth Layer (medium-sensitivity red-sensitive emulsion layer) | |
|---|---|
| Silver Iodobromide Emulsion D as silver | 0.70 |
| ExS-1 | 3.0×10⁻⁴ |
| ExS-2 | 1.2×10⁻⁵ |
| ExS-3 | 4.0×10⁻⁴ |
| ExC-1 | 0.22 |
| ExC-2 | 0.055 |
| ExC-5 | 0.007 |
| ExC-8 | 0.009 |
| Cpd-2 | 0.036 |
| HBS-1 | 0.11 |
| Gelatin | 0.70 |

| First Layer (antihalation layer) | |
|---|---|
| Black colloidal silver as silver | 0.18 |
| Gelatin | 1.60 |
| ExM-1 | 0.11 |
| ExF-1 | 3.4×10⁻³ |
| ExF-2 (solid disperse dye) | 0.03 |
| ExF-3 (solid disperse dye) | 0.04 |
| HBS-1 | 0.16 |

| Third Layer (low-sensitivity red-sensitive emulsion layer) | |
|---|---|
| Silver Iodobromide Emulsion A as silver | 0.46 |
| ExS-1 | 5.0×10⁻⁴ |
| ExS-2 | 1.8×10⁻⁵ |
| ExS-3 | 5.0×10⁻⁴ |
| ExC-1 | 0.11 |
| ExC-3 | 0.045 |
| ExC-5 | 0.0050 |
| ExC-7 | 0.001 |
| ExC-8 | 0.010 |
| Cpd-2 | 0.005 |
| HBS-1 | 0.090 |
| Gelatin | 0.87 |

| Seventh Layer (low-sensitivity green-sensitive emulsion layer) | |
|---|---|
| Silver Iodobromide Emulsion A as silver | 0.24 |
| Silver Iodobromide Emulsion B as silver | 0.10 |
| Silver Iodobromide Emulsion C as silver | 0.14 |
| ExS-4 | 4.0×10⁻⁵ |
| ExS-5 | 1.8×10⁻⁴ |
| ExS-6 | 6.5×10⁻⁴ |
| ExM-1 | 0.005 |
| ExM-2 | 0.30 |
| ExM-3 | 0.09 |
| ExY-1 | 0.015 |
| HBS-1 | 0.26 |
| HBS-3 | 0.006 |
| Gelatin | 0.80 |

| Eighth Layer (medium-sensitivity green-sensitive emulsion layer) | |
|---|---|
| Silver Iodobromide Emulsion D as silver | 0.94 |
| ExS-4 | 2.0×10 ⁻⁵ |
| ExS-5 | 1.4×10 ⁻⁴ |
| ExS-6 | 5.4×10 ⁻⁴ |
| ExM-2 | 0.16 |
| ExM-3 | 0.045 |
| ExY-1 | 0.008 |
| ExY-5 | 0.030 |
| HBS-1 | 0.14 |
| HBS-3 | 8.0×10 ⁻³ |
| Gelatin | 0.90 |

| Fifth Layer (high-sensitivity red-sensitive emulsion layer) | |
|---|---|
| Silver Iodobromide Emulsion E as silver | 1.62 |
| ExS-1 | 2.0×10⁻⁴ |
| ExS-2 | 1.0×10⁻⁵ |
| ExS-3 | 3.0×10⁻⁴ |
| ExC-1 | 0.133 |
| ExC-3 | 0.040 |
| ExC-6 | 0.040 |
| ExC-8 | 0.014 |
| Cpd-2 | 0.050 |
| HBS-1 | 0.22 |
| HBS-2 | 0.10 |
| Gelatin | 0.85 |

| Tenth Layer (yellow filter layer) | |
|---|---|
| Yellow colloidal silver as silver | 0.010 |
| Cpd-1 | 0.10 |
| ExF-5 (solid disperse dye) | 0.06 |
| ExF-6 (solid disperse dye) | 0.06 |
| ExF-7 (oil-soluble dye) | 0.005 |
| HBS-1 | 0.055 |
| Gelatin | 0.70 |

| Seventh Layer (low-sensitivity green-sensitive emulsion layer) | |
|---|---|
| Silver Iodobromide Emulsion A as silver | 0.24 |
| Silver Iodobromide Emulsion B as silver | 0.10 |
| Silver Iodobromide Emulsion C as silver | 0.14 |
| ExS-4 | 4.0×10⁻⁵ |
| ExS-5 | 1.8×10⁻⁴ |
| ExS-6 | 6.5×10⁻⁴ |
| ExM-1 | 0.005 |
| ExM-2 | 0.30 |
| ExM-3 | 0.09 |
| ExY-1 | 0.015 |
| HBS-1 | 0.26 |
| HBS-3 | 0.006 |
| Gelatin | 0.80 |

| Ninth Layer (high-sensitivity green-sensitive emulsion layer) | |
|---|---|
| Silver Iodobromide Emulsion E as silver | 1.29 |
| ExS-4 | 3.7×10⁻⁵ |
| ExS-5 | 8.1×10⁻⁵ |
| ExS-6 | 3.2×10⁻⁴ |
| ExC-4 | 0.011 |
| ExM-1 | 0.016 |
| ExM-4 | 0.046 |
| ExM-5 | 0.023 |
| Cpd-3 | 0.050 |
| HBS-1 | 0.20 |
| HBS-2 | 0.08 |
| Polyethyl acrylate latex | 0.26 |
| Gelatin | 0.82 |

| Thirteenth Layer (first protective layer) | |
|---|---|
| UV-2 | 0.08 |
| UV-3 | 0.11 |
| UV-4 | 0.26 |
| HBS-1 | 0.09 |
| Gelatin | 1.20 |

| Fourteenth Layer (second protective layer) | |
|---|---|
| Silver Iodobromide Emulsion G as silver | 0.10 |
| H-1 | 0.30 |
| B-1 (diameter: 1.7 µm) | 5.0×10⁻² |
| B-2 (diameter: 1.7 µm) | 0.10 |
| B-3 | 0.10 |
| S-1 | 0.20 |
| Gelatin | 1.75 |

In addition, to each layer, W-1 to W-3, B-4 to B-6, F-1 to F-17, an iron salt, a lead salt, a gold salt, a platinum salt, an iridium salt, a palladium salt and a rhodium salt were appropriately added to improve preservability, processability, pressure durability, antimold and bactericidal property, antistatic property and coatability.

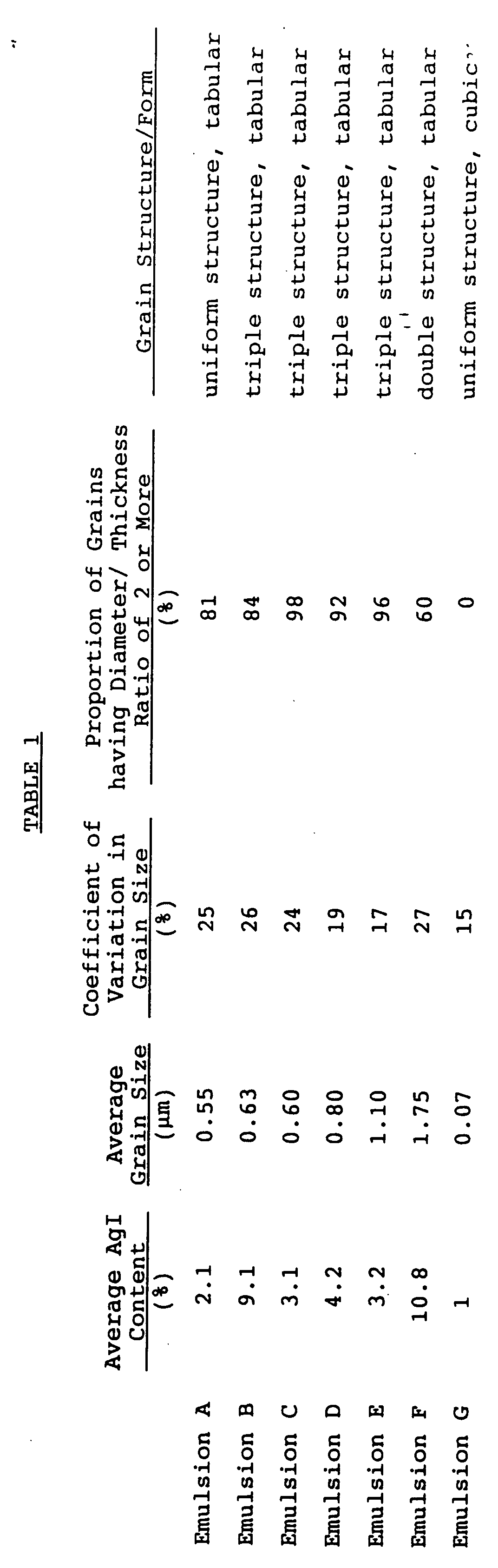

In Table 1:
(1) Emulsions A to F were subjected to reduction sensitization at the grain preparation using thiourea dioxide and thiosulfonic acid according to the example of JP-A-2-191938;
(2) Emulsions A to F were subjected to gold sensitization, sulfur sensitization and selenium sensitization in the presence of the spectral sensitizing dyes described in each light-sensitive layer and sodium thiocyanate according to the example of JP-A-3-237450;
(3) in the preparation of tabular grains, low molecular weight gelatin was used according to the example of JP-A-1-158426; and
(4) in tabular grains, dislocation lines were observed through a high-pressure electron microscope as described in JP-A-3-237450.

### Preparation of Dispersion of Organic Solid Disperse Dye:

ExF-2 shown below was dispersed in the following manner. Namely, 21.7 ml of water, 3 ml of a 5% aqueous solution of sodium p-octylphenoxyethoxyethoxyethanesulfonate and 0.5 g of a 5% aqueous solution of p-octylphenoxypolyoxyethylene ether (polymerization degree: 10) were poured in a 700 ml-volume pot mill, then thereto 5.0 g of Dye ExF-2 and 500 ml of zirconium oxide beads (diameter: 1 mm) were added and the content was dispersed for 2 hours. The dispersion was conducted using a BO-type vibrating ball mill manufactured by Chuo Koki K.K. After the dispersion, the content was taken out and added to 8 g of a 12.5% aqueous gelatin solution and beads were removed by filtration to obtain a gelatin dispersion of the dye. The fine dye particles had an average particle diameter of 0.44 µm.

In the same manner, solid dispersions of ExF-3, ExF-4 and ExF-6 were obtained. The fine dye particles had an average particle diameter of 0.24 µm, 0.45 µm and 0.52 µm, respectively. ExF-5 was dispersed by the microprecipitation dispersion method described in Example 1 of European Unexamined Patent Publication (EP) 0549489A. The average particle diameter thereof was 0.06 µm.

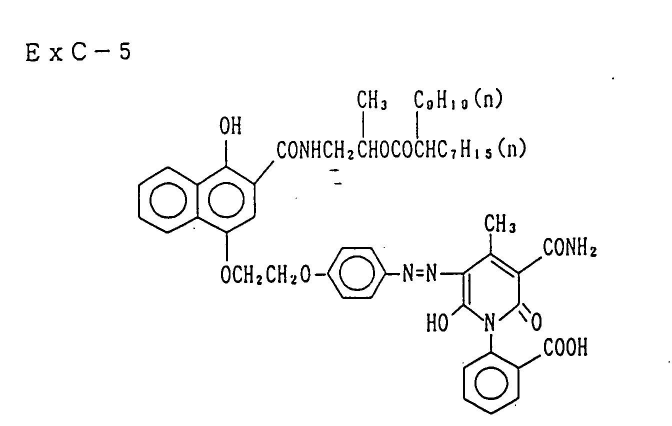

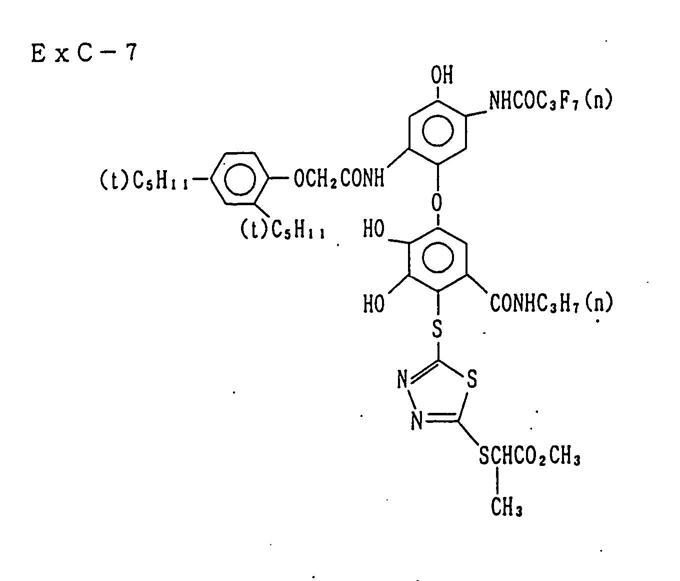

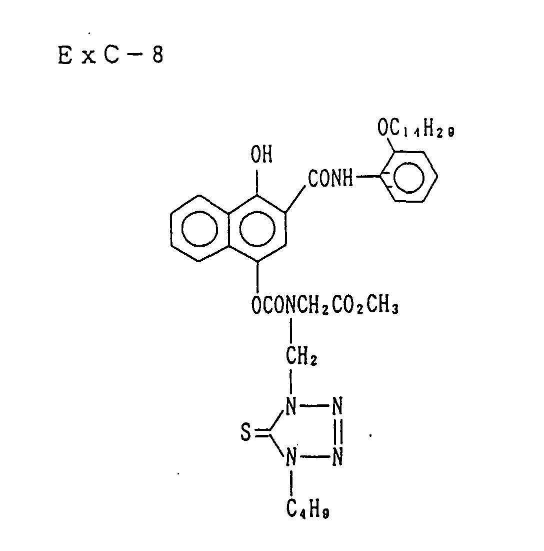

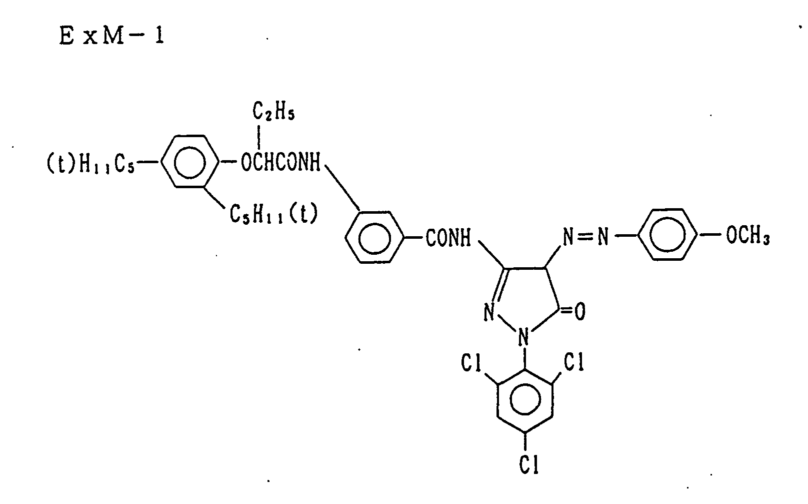

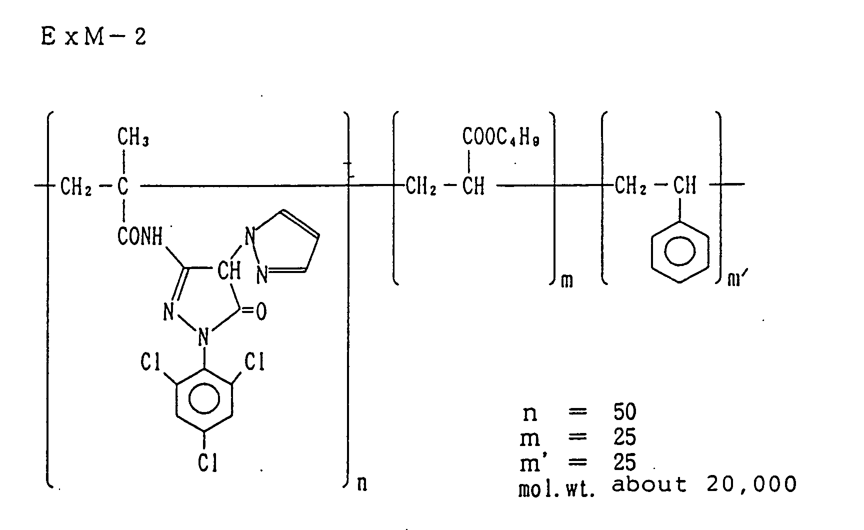

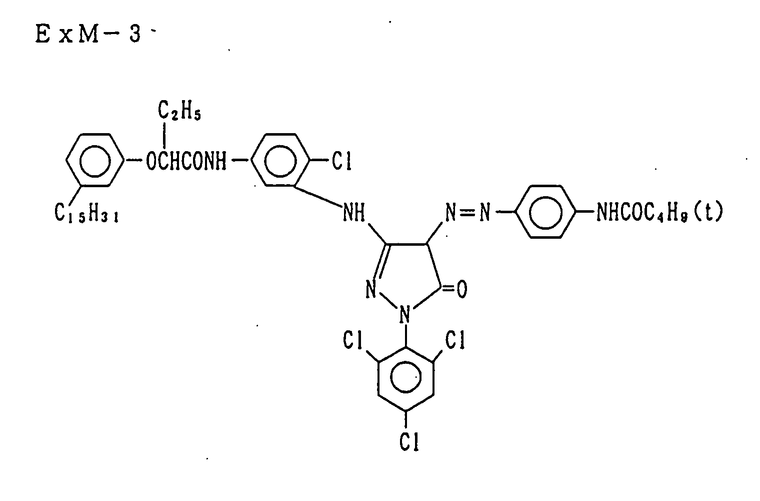

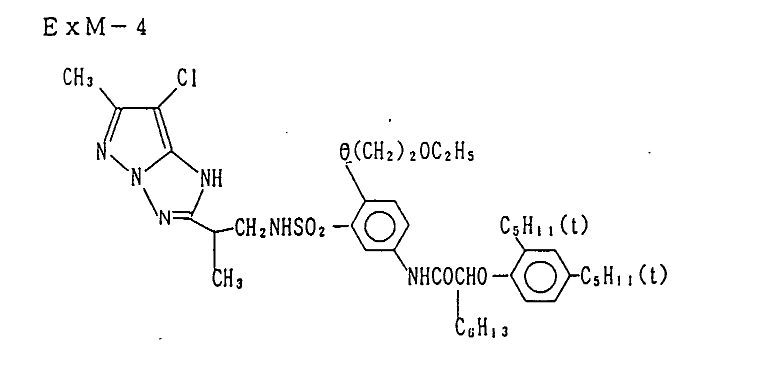

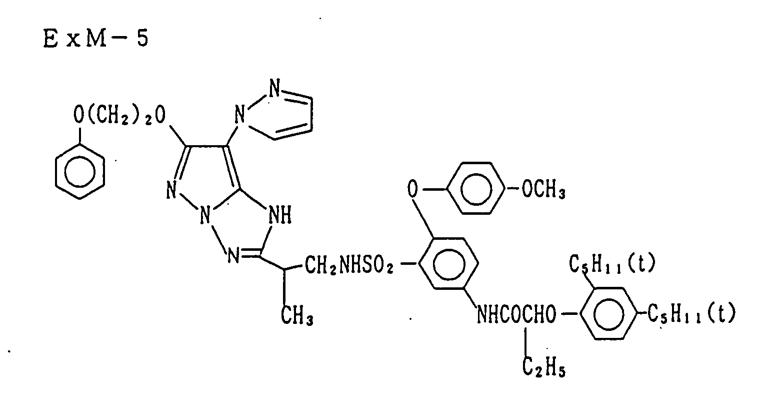

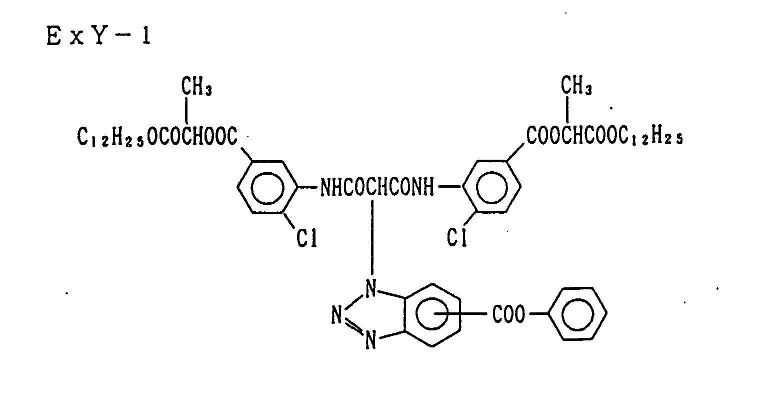

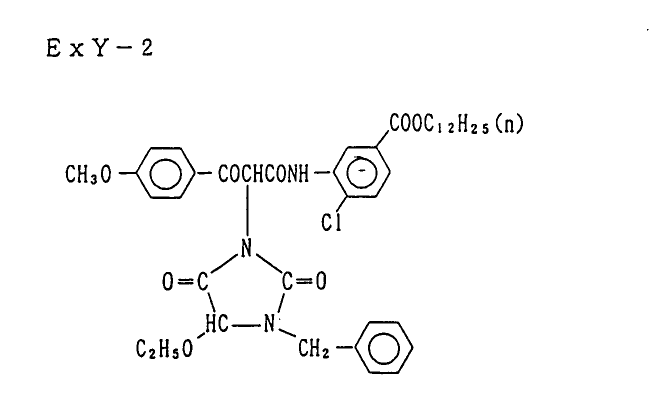

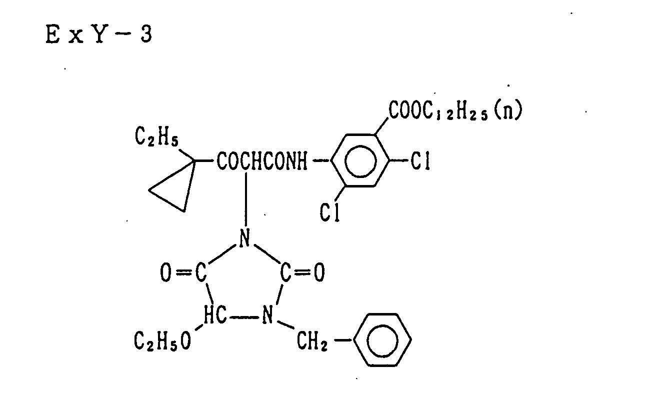

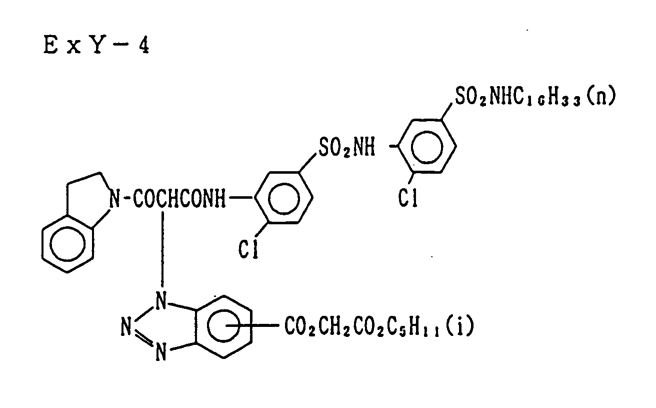

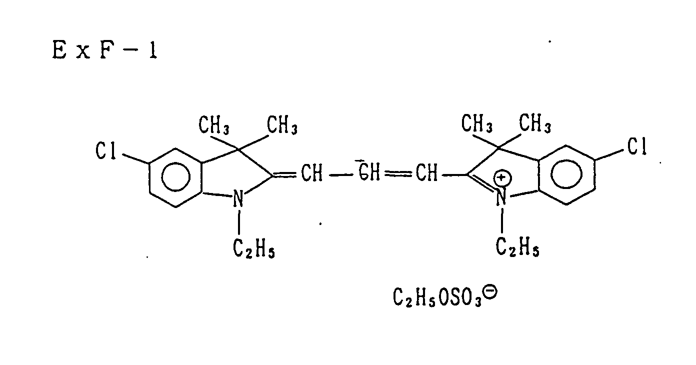

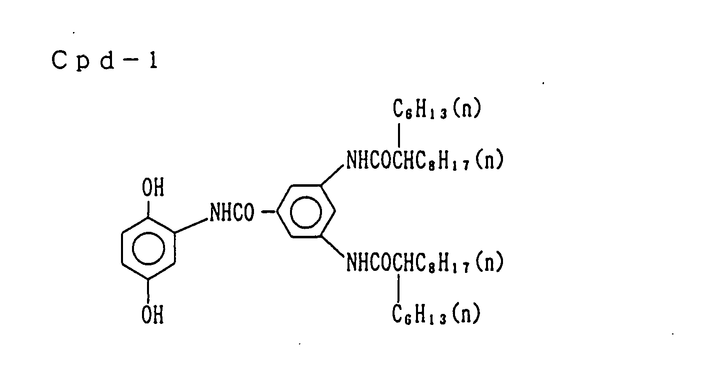

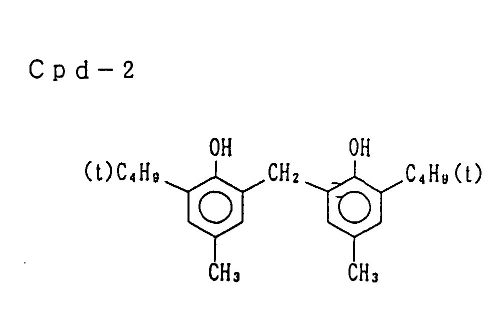

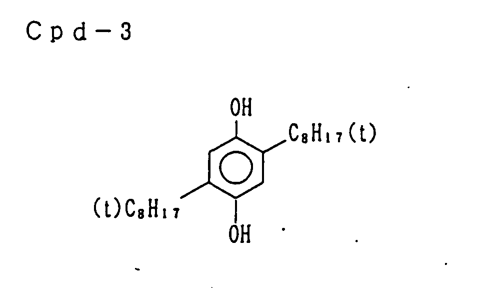

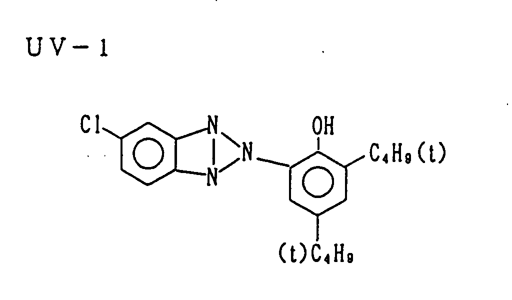

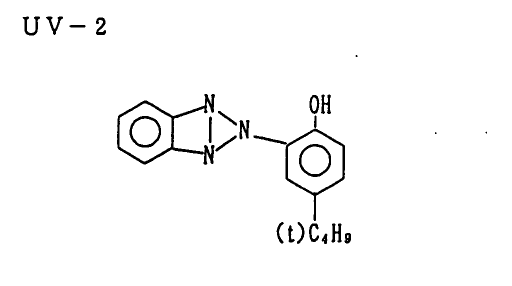

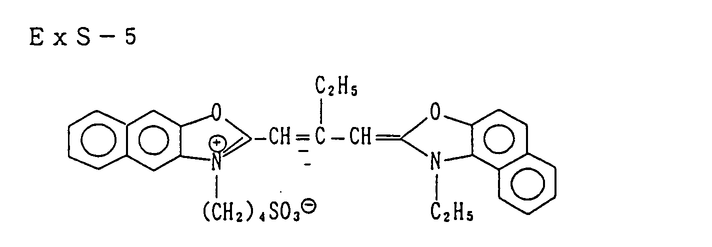

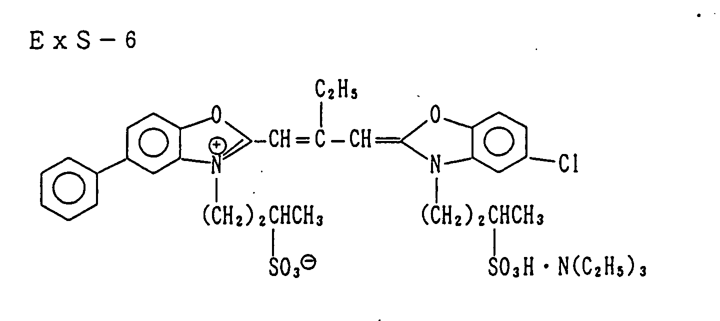

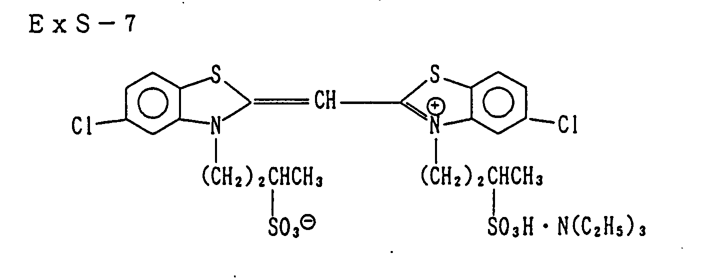

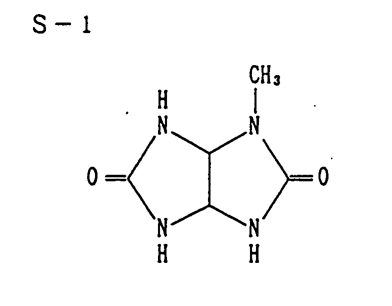

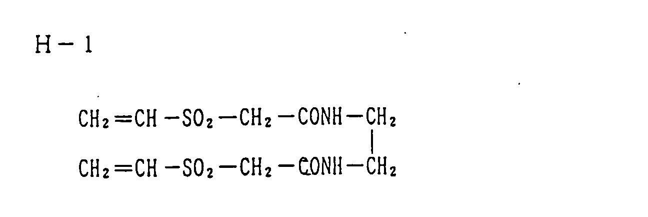

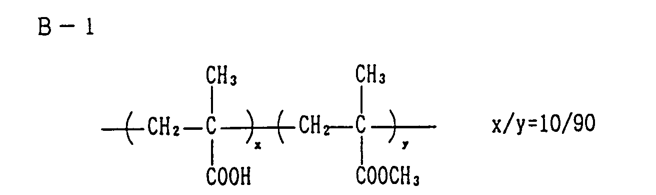

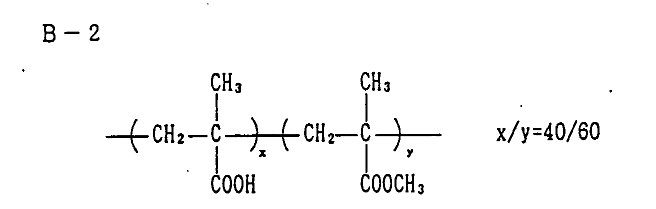

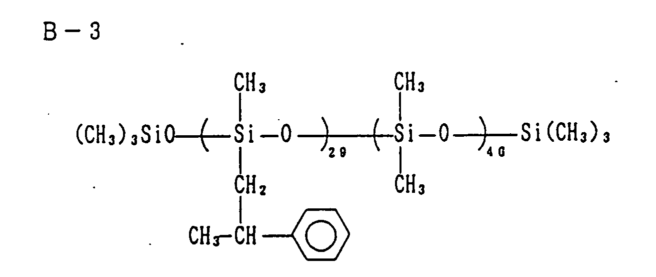

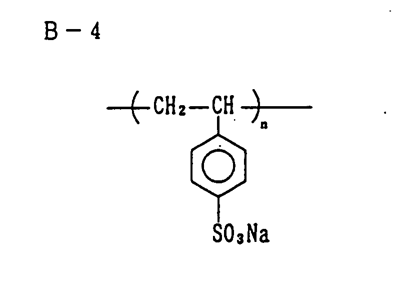

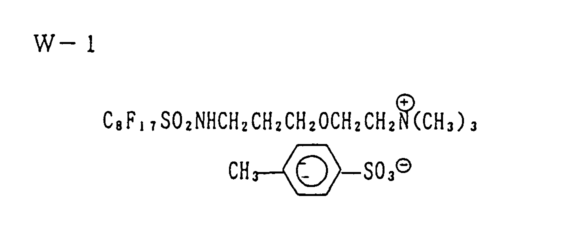

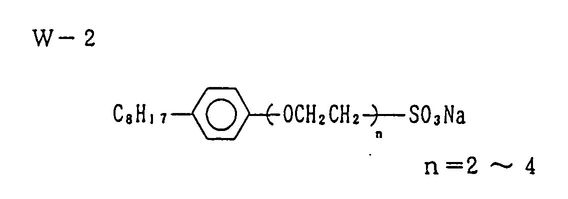

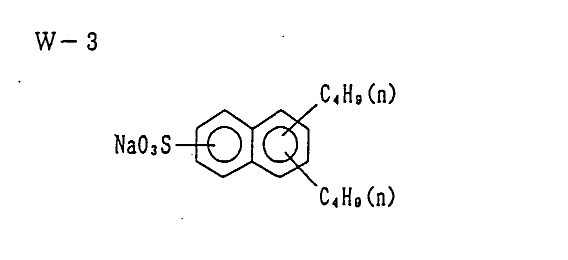

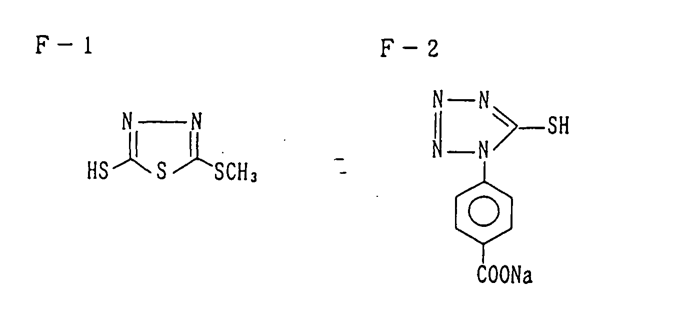

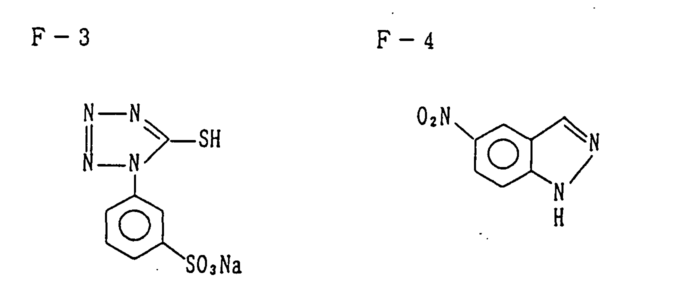

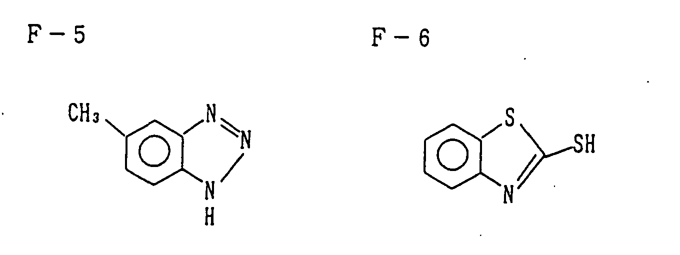

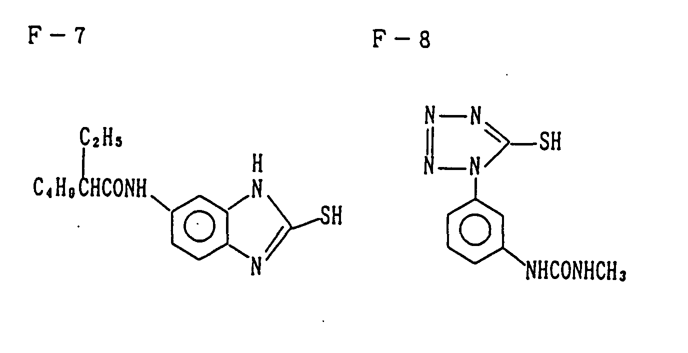

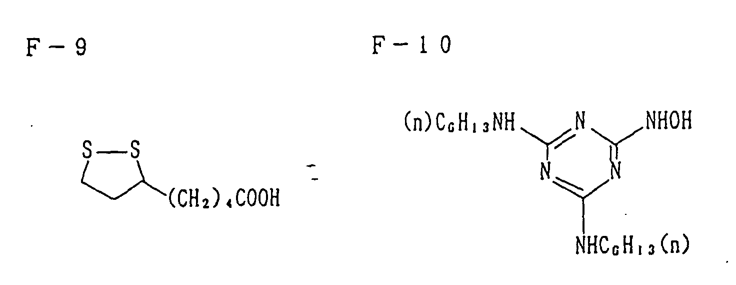

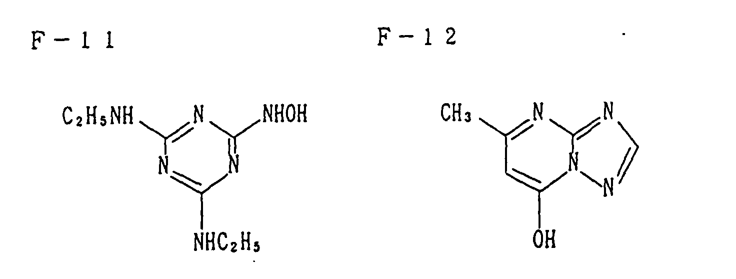

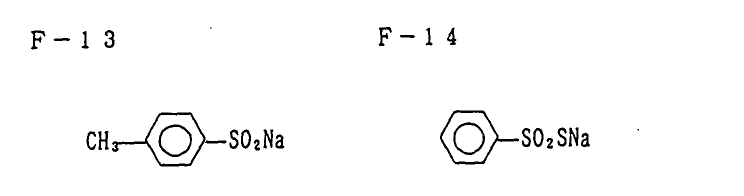

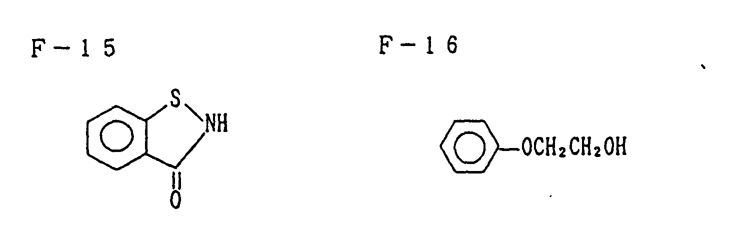

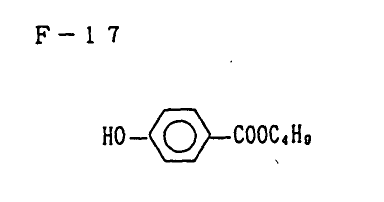

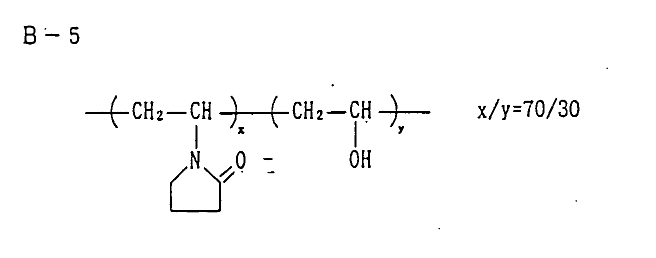

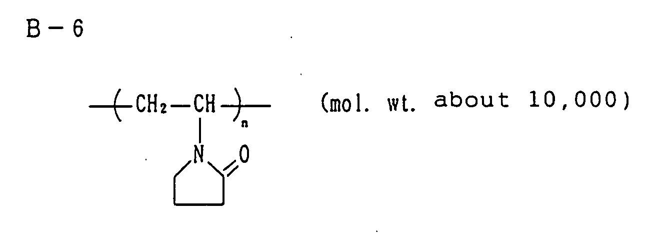

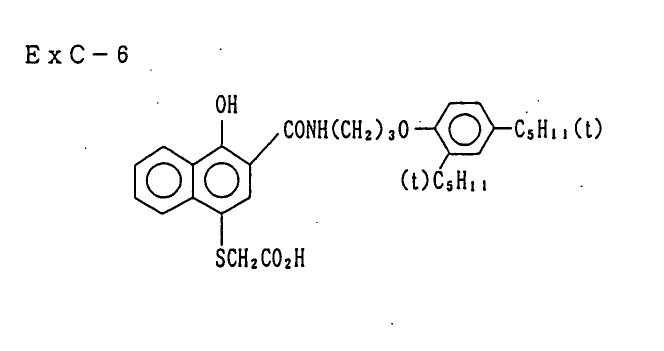

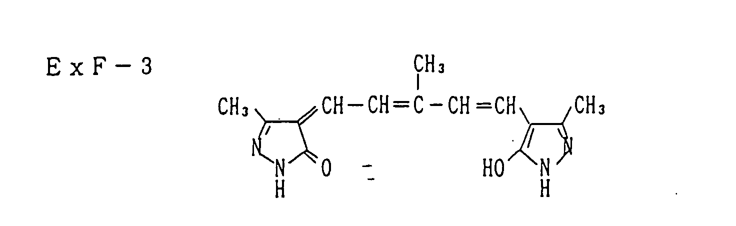

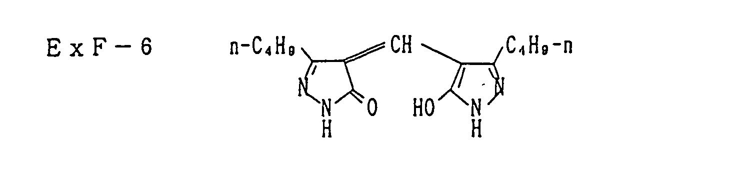

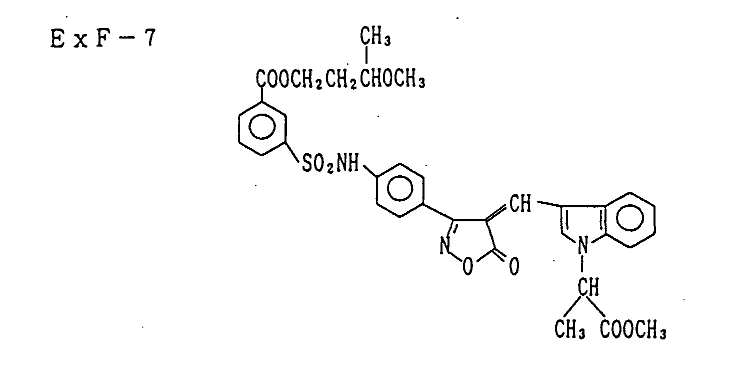

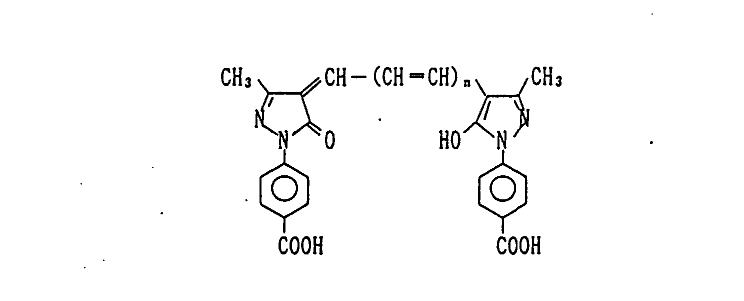

ExF-2 (n=2)

ExF-4 (n=1)

ExF-5 (n=0)

Sample 101 prepared above and cut into a 35-mm width was imagewise exposed and processed using the processing solution shown below in an automatic developing machine FP-560B manufactured by Fuji Photo Film Co., Ltd.

In order to clarify the level achieved on the overcoming of problems intended to be solved by the compound used in the present invention, the state was made the same as that when continuous processing was conducted forcedly. Namely, 100 ml of the bleaching solution prepared according to the following formulation was added to each of the color developing bath and the fixing bath and stirred for 5 hours while keeping the processing temperature, and then the processing was conducted.

In order to examine the change in the bleaching capability depending on the concentration, the processing was conducted by varying the iron chelate concentration in the bleaching solution. The bleaching solution 1 was prepared to have an iron chelate concentration of 240 mM and the bleaching solution 2 was prepared to have an iron chelate concentration of 120 mM so as to examine the capability at a diluted concentration.

The processing steps and the processing compositions are shown below.

### (Processing Steps)

| Step | Processing Time | Processing Temperature (°C) | Tank Volume (ℓ) |
|---|---|---|---|
| Color development | 3 min 5 s | 38.0 | 17 |
| Bleaching (using bleaching solution 1 or 2) | 2 min | 38.0 | 5 |
| Fixing | 2 min | 38.0 | 5 |
| Water washing | 30 s | 38.0 | 3.5 |
| Stabilization (1) | 20 s | 38.0 | 3 |
| Stabilization (2) | 20 s | 38.0 | 3 |
| Drying | 1 min 30 s | 60 | |
| * The replenishing amount was per 1.1 m of the 35 mm-width light-sensitive material (corresponding to one roll of 24 Ex .) | | | |

The crossover time between respective processing steps was 6 seconds and it was included in the processing time of the previous step.

### (Composition of Processing Solution)

### (Color Developer)

| | Tank Solution (g) |
|---|---|
| Diethylenetriaminepentaacetic acid | 2.0 |
| 1-Hydroxyethylidene-1,1-diphosphonic acid | 2.0 |
| Sodium sulfite | 3.9 |
| Potassium carbonate | 37.5 |
| Potassium bromide | 1.4 |
| Potassium iodide | 1.3 mg |
| Hydroxylamine sulfate | 2.4 |
| 2-Methyl-4-[N-ethyl-N-(β-hydroxyethyl)amino]aniline sulfate | 4.5 |
| Water to make | 1.0 ℓ |
| pH (adjusted with potassium hydroxide and sulfuric acid) | 10.05 |

### (Bleaching Solution 1)

| | Tank Solution (g) |
|---|---|
| Iron nitrate nonahydrate | 0.24 mol |
| Chelate compound | 0.26 mol |
| Ammonium bromide | 70 |
| Ammonium nitrate | 14 |
| Hydroxyacetic acid | 50 |
| Acetic acid | 40 |
| Water to make | 1.0 ℓ |
| pH (adjusted by aqueous ammonia) | 4.0 |

| | Tank Solution (g) |
|---|---|
| Iron nitrate nonahydrate | 0.12 mol |
| Chelate compound | 0.13 mol |
| Ammonium bromide | 70 |
| Ammonium nitrate | 14 |
| Hydroxyacetic acid | 50 |
| Acetic acid | 40 |
| Water to make | 1.0 ℓ |
| pH (adjusted by aqueous ammonia) | 4.0 |

### (Fixing Solution)

| | Tank Solution (g) |
|---|---|
| Ammonium sulfite | 19 |
| Aqueous solution of ammonium thiosulfate (700 g/ℓ) | 280 ml |
| Imidazole | 15 |
| Ethylenediaminetetraacetic acid | 15 |
| Water to make | 1.0 ℓ |
| pH (adjusted with aqueous ammonia and acetic acid) | 7.0 |

### (Washing Water)

Tap water was passed through a mixed bed column filled with an H-type strongly acidic cation exchange resin (Amberlite IR-120B, produced by Rhom & Haas) and an OH-type strongly basic anion exchange resin (Amberlite IR-400, produced by the same company) to reduce the calcium and magnesium ion concentrations each to 3 mg/liter or less and then thereto 20 mg/liter of dichlorinated sodium isocyanurate and 150 mg/liter of sodium sulfate were added. The resulting solution had a pH of from 6.5 to 7.5.

### (Stabilizing Solution)

| | Tank Solution (unit: g) |
|---|---|
| Sodium p-toluenesulfinate | 0.03 |
| Polyoxyethylene-p-monononylphenyl ether (average polymerization degree: 10) | 0.2 |
| Disodium ethylenediaminetetraacete | 0.05 |
| 1,2,4-Triazole | 1.3 |
| 1,4-Bis(1,2,4-triazol-1-ylmethyl)piperazine | 0.75 |
| Water to make | 1.0 ℓ |
| pH | 8.5 |

The thus-processed multi-layer color photographic Sample 101 was measured on the residual silver at the maximum density area according to fluorescent X-ray analysis. The results obtained are shown in Table 2. Further, the sample obtained after the processing was measured with green light (G light) and Dmin value was read out.

Then, as a standard processing method having no bleaching fog, the bleaching step in the above-described processing was changed to replace the formulation of the processing solution as follows. Other than those described below were not changed.

| Step | Processing Time | Processing Temperature |
|---|---|---|
| Bleaching | 3 min 00 s | 38°C |

### (Standard Bleaching Solution)

| | Tank Solution |
|---|---|
| | (g) |
| Sodium ethylenediaminetetraacetato | 100.0 |
| ferrate complex salt trihydrate | |
| Disodium ethylenediaminetetraacetate | 10.0 |
| 3-Mercapto-1,2,4-triazole | 0.03 |
| Ammonium bromide | 140.0 |
| Ammonium nitrate | 30.0 |
| Aqueous ammonia (27%) | 6.5 ml |

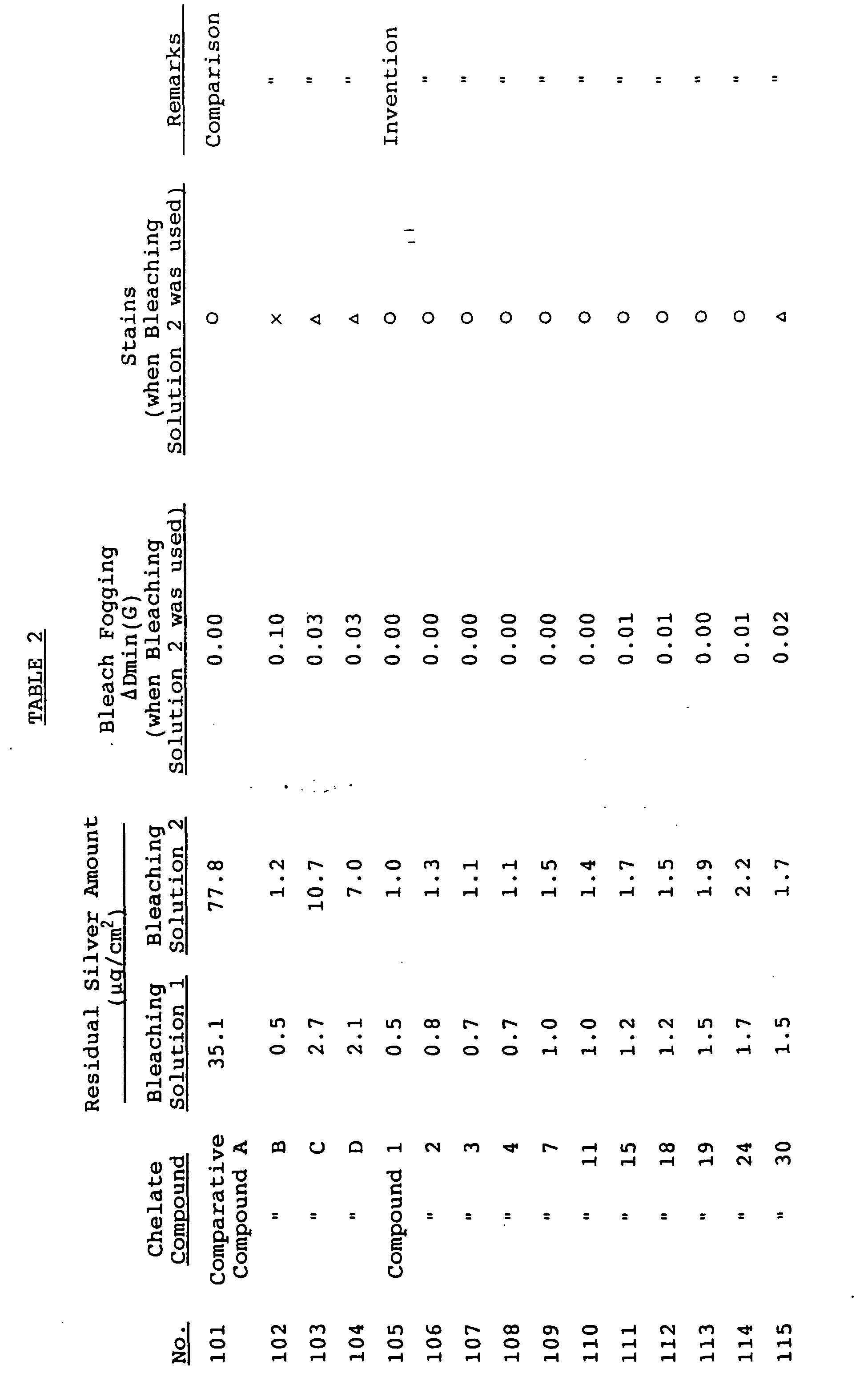

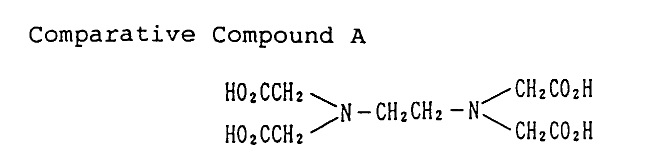

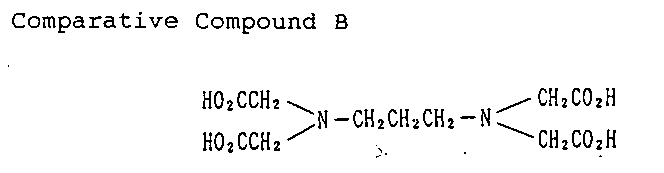

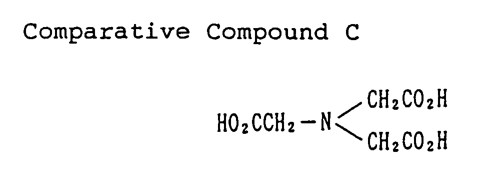

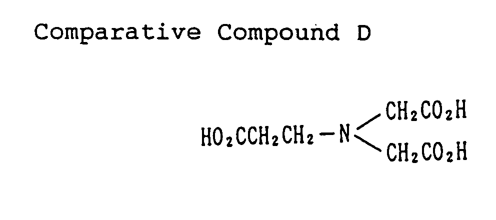

As seen in Table 2, the present invention provided satisfactory results overall with respect to the desilvering property at a diluted concentration, the bleaching fog when the bleaching solution mingled into the color developing bath and the generation of stains when the bleaching solution mingled into the fixing bath, and the superiority of present invention was verified.

### EXAMPLE 2

A multi-layer color photographic printing paper described in Example 4 of JP-A-5-303186 (Sample 001) and the following processing solution were prepared.

### (Color Developer)

| | Tank Solution (g) | Replenisher (g) |
|---|---|---|
| Water | 700 ml | 700 ml |
| Diethylenetriaminepentaacetic acid | 0.4 g | 0.4 g |
| N,N,N-Tris(methylenephosphonic acid) | 4.0 g | 4.0 g |
| Disodium 1,2-dihydroxybenzene-4,6-disulfonate | 0.5 g | 0.5 g |
| Triethanol amine | 12.0 g | 12.0 g |
| Potassium chloride | 6.5 g | - |
| Potassium bromide | 0.03 g | - |
| Potassium carbonate | 27.0 g | 27.0 g |
| Aminostilbene-base fluorescent brightening agent (WHITEX 4, produced by Sumitomo Chemical Company, Limited) | 1.0 g | 3.0 g |
| Sodium sulfite | 0.1 g | 0.1 g |
| N,N-bis(sulfoethyl)hydroxylamine | 10.0 g | 13.0 g |
| N-Ethyl-N-(β-methanesulfonamidoethyl)-3-methyl-4-aminoaniline sulfate | 5.0 g | 11.5 g |
| Water to make | 1,000 ml | 1,000 ml |
| pH (at 25°C) | 10.10 | 11.10 |

### (Bleach-fixing Solution)

| | Tank Solution (g) | Replenisher (g) |
|---|---|---|
| Water | 600 ml | 600 ml |
| Ammonium thiosulfate (700 g/ℓ) | 100 ml | 250 ml |
| Ammonium sulfite | 40 g | 100 g |
| Chelate compound (shown in Table 3) | 0.166 mol | 0.407 mol |
| Iron nitrate nonahydrate | 0.138 mol | 0.339 mol |
| Ethylenediaminetetraacetic acid | 5 g | 12.5 g |
| Ammonium bromide | 40 g | 75 g |
| Nitric acid (67%) | 30 g | 65 g |
| Water to make | 1,000 ml | 1,000 ml |
| pH (at 25°C) (adjusted by acetic acid and aqueous ammonia) | 6.5 | 6.3 |

### (Rinsing Solution)

The tank solution and the replenisher were common. Tap water was passed through a mixed bed column filled with an H-type strongly acidic cation exchange resin (Amberlite IR-120B, produced by Rhom & Haas) and an OH-type strongly basic anion exchange resin (Amberlite IRA-400, produced by the same company) to reduce the calcium and magnesium ion concentrations each to 3 mg/liter or less and then thereto 20 mg/liter of dichlorinated sodium isocyanurate and 150 mg/liter of sodium sulfate were added. The resulting solution had a pH of from 6.5 to 7.5.

In order to examine the residual silver amount after the processing, the multi-layer color photographic printing paper (Sample 001) was uniformly exposed to give a gray density of 2.2 and then processed through the following processing steps. The residual silver amount was measured by fluorescent X-ray analysis. Further, in order to examine the increase in stains after the processing, the sample was subjected to gradation exposure and then processed in the same manner. The processed sample was aged at 80°C and 70% for one week and the increase in stains between before and after the aging was examined. The processing was started with the above-described processing solutions by pouring the tank solution in each step into each processing tank and continued while adding the replenisher to each tank according to the amount processed. The processing was conducted until the cumulative replenishing amount reached 3 times the tank volume and the results of the processing conducted at this time are shown in Table 3.

### (Processing Step)

| Step | Temperature (°C) | Time (s) | Replenishing Amount* (ml) | Tank Volume (ℓ) |
|---|---|---|---|---|
| Color development | 39 | 45 | 70 | 20 |
| Bleach-fixing | 35 | (1) 45 | 60** | 20 |
| | | (2) 20 | | |
| Rinsing (1) | 35 | 20 | - | 10 |
| Rinsing (2) | 35 | 20 | - | 10 |
| Rinsing (3) | 35 | 20 | 360 | 10 |
| Drying | 80 | 60 | | |

| | | | | |
|---|---|---|---|---|
| (* Replenishing amount per m² of the light-sensitive material) (Rinsing was in a three-tank countercurrent system from Rinsing (3) to Rinsing (1).) | | | | |
| (** In addition to 60 ml above, 120 ml was flown thereinto from Rinsing (1) per m² of the light-sensitive material.) | | | | |

**TABLE 3**

| No. | Chelate Compound | Bleach-Fixing Time | Residual Silver Amount (µg/cm²) | Remarks |
|---|---|---|---|---|
| 201 | Comparative Compound A | 45 | 2.7 | Comparison |
| | | 20 | 8.3 | |
| 202 | Comparative Compound B | 45 | 10.9 | " |
| | | 20 | 21.1 | |
| 203 | Comparative Compound C | 45 | 2.5 | " |
| | | 20 | 7.9 | |
| 204 | Comparative Compound D | 45 | 2.2 | " |
| | | 20 | 7.0 | |
| 205 | Compound 1 | 45 | 0.5 | Invention |
| | | 20 | 1.3 | |
| 206 | Compound 2 | 45 | 0.7 | " |
| | | 20 | 1.5 | |
| 207 | Compound 3 | 45 | 0.5 | " |
| | | 20 | 1.2 | |
| 208 | Compound 7 | 45 | 1.0 | " |
| | | 20 | 2.1 | |
| 209 | Compound 18 | 45 | 1.2 | " |
| | | 20 | 2.5 | |
| 210 | Compound 19 | 45 | 1.1 | " |
| | | 20 | 2.3 | |
| 211 | Compound 24 | 45 | 1.5 | " |
| | | 20 | 2.8 | |

Comparative Compounds A, B, C and D were the same as used in Example 1.

As shown in Fig. 3, the metal chelate compounds used in the present invention were superior to comparative compounds in the desilvering property even when the processing solution having bleaching ability was used as the bleach-fixing bath of a multilayer color photographic printing paper. In particular, this effect was outstanding in the processing where the bleach-fixing time was reduced. Namely, even when the bleach-fixing time was reduced to a half, the residual silver amount was small before and after the running, revealing excellent desilvering property.

The present invention is excellent in the desilvering property, the bleaching fog and the running stability.

## Claims

1. A processing composition for a silver halide photographic material containing at least one metal chelate compound which acts as an oxidising agent for a silver halide photographic material the chelate compound being represented by the formula (I) 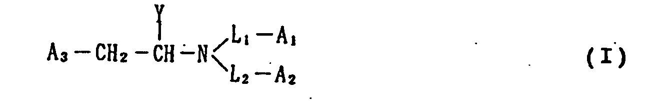 wherein Y represents an optionally substituted cyclic aliphatic hydrocarbon group, a 3- to 10-membered unsaturated or saturated heterocyclic group having at least one of N, O and S atoms which may be monocyclic or may be condensed with another ring to form a condensed ring, or an optionally substituted aryl group; L₁ and L₂ each represents an optionally substituted alkylene group; and A₁, A₂ and A₃ each represents a carboxyl group, a phosphono group, a sulfo group or a hydroxy group,
provided that the processing composition is not a processing solution having a silver bleaching ability comprising a ferric complex salt of 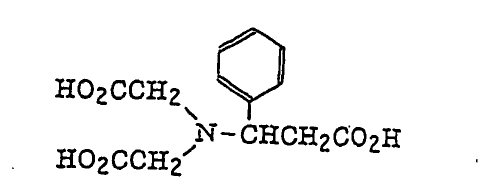 and either a persulfate or a compound of formula 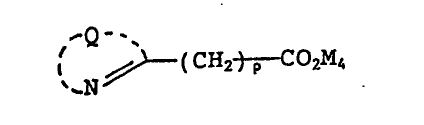 wherein Q represents a nonmetallic atomic group necessary for forming a nitrogen-containing heterocyclic ring; p represents 0 or 1; and M₄ represents a hydrogen atom or a cation.

2. The processing composition of claim 1, wherein the compound represented by formula (I) is represented by formula (III) 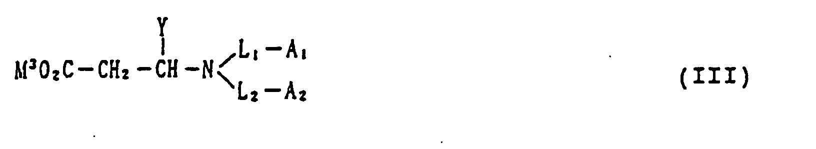 wherein Y, L₁, L₂, A₁ and A₂ have the same meaning as those in formula (I), and M³ represents a hydrogen atom or a cation.

3. The processing composition of claim 1, wherein the compound represented by formula (I) is represented by formula (IV) 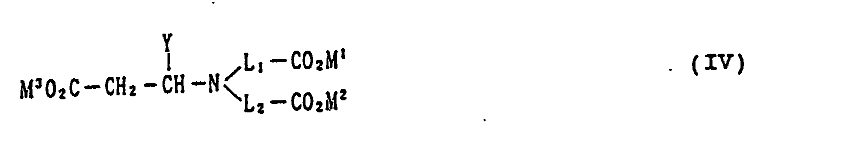 wherein Y, L₁, L₂, A₁ and A₂ have the same meaning as those in formula (I), and M¹, M² and M³ each represents a hydrogen atom or a cation.

4. The processing composition of claim 1, wherein the compound represented by formula (I) is represented by formula (V) 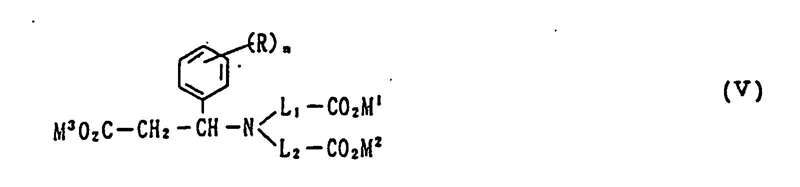 wherein L₁ and L₂ have the same meaning as those in formula (I); M¹, M² and M³ each represents a hydrogen atom or a cation; R represents a substituent; and n represents an integer of from 0 to 5.

5. The processing composition of claim 1, which is a bleaching solution or a bleach-fixing solution.

6. The processing composition of claim 1, which contains the metal chelate compound in an amount of from 0.005 to 1 mol/l.

7. The processing composition of claim 1, wherein the compound of formula (I) is a bleaching agent.

8. A method for processing a silver halide photographic material comprising processing an imagewise exposed silver halide photographic material with the processing composition of any of claims 1 to 7.

## Patentansprüche

1. Verarbeitungszusammensetzung für ein photographisches Silberhalogenidmaterial, enthaltend mindestens eine Metallcheiatverbindung, die als Oxidationsmittel für ein photographisches Silberhalogenidmaterial dient, wobei die Chelatverbindung durch die Formel (I) dargestellt wird 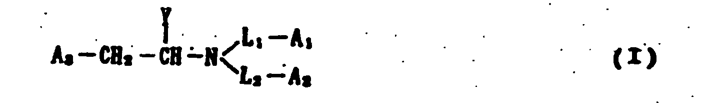 worin Y eine gegebenenfalls substituierte cyclische aliphatische Kohlenwasserstoffgruppe, eine 3- bis 10-gliedrige ungesättigte oder gesättigte heterocyclische Gruppe mit mindestens einem Heteroatom, ausgewählt aus N, O und S, die eine monocyclische Gruppe sein kann oder die mit einem anderen Ring kondensiert sein kann, wobei ein kondensierter Ring gebildet wird, oder eine gegebenenfalls substituierte Arylgruppe bedeutet; L₁ und L₂ bedeuten jeweils eine gegebenenfalls substituierte Alkylengruppe; und A₁, A₂ und A₃ bedeuten jeweils eine Carboxygruppe, eine Phosphonogruppe, eine Sulfogruppe oder eine Hydroxygruppe, mit der Maßgabe, dass die Verarbeitungszusammensetzung keine Verarbeitungslösung ist, die Silber bleichen kann und die ein Eisen(III)komplexsalz von 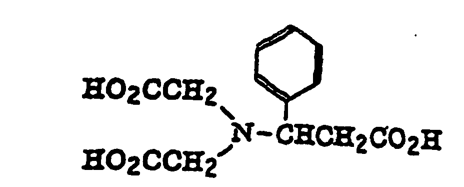 und entweder ein Persulfat oder eine Verbindung der Formel 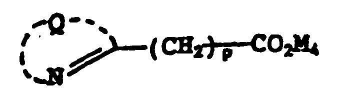 umfasst, worin Q eine Gruppe von Nichtmetallatomen ist, die erforderlich ist, um einen Stickstoff enthaltenden heterocydischen Ring zu bilden; p bedeutet 0 oder 1; und M₄ bedeutet ein Wasserstoffatom oder ein Kation.

2. Verarbeitungszusammensetzung nach Anspruch 1, wobei die Verbindung, die durch die Formel (I) dargestellt wird, eine Verbindung ist, die durch die Formel (III) dargestellt wird 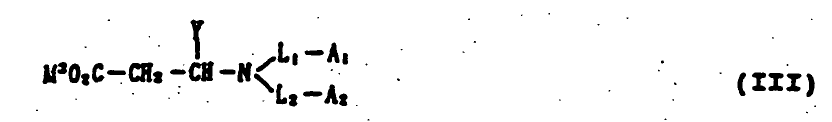 worin Y, L₁, L₂, A₁ und A₂ die gleiche Bedeutung wie in Formel (I) haben, und M³ bedeutet ein Wasserstoffatom oder ein Kation.

3. Verarbeitungszusammensetzung nach Anspruch 1, wobei die Verbindung, die durch die Formel (I) dargestellt wird, eine Verbindung ist, die durch die Formel (IV) dargestellt wird 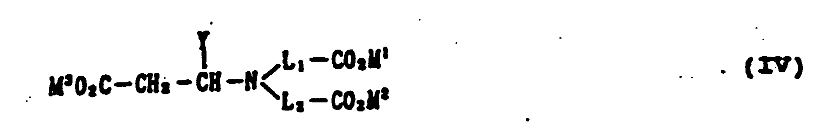 worin Y, L₁, L₂, A₁ und A₂ die gleiche Bedeutung wie in Formel (1) haben, und M¹, M² und M³ bedeuten jeweils ein Wasserstoffatom oder ein Kation.

4. Verarbeitungszusammensetzung nach Anspruch 1, wobei die Verbindung, die durch die Formel (I) dargestellt wird, eine Verbindung ist, die durch die Formel (V) dargestellt wird 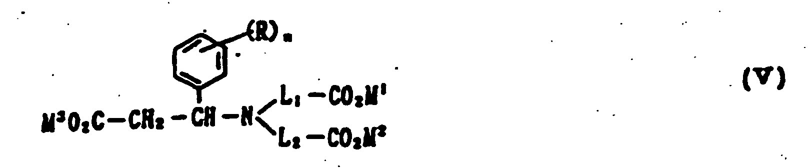 worin L₁ und L₂ die gleiche Bedeutung wie in Formel (I) haben; M¹, M² und M³ bedeuten jeweils ein Wasserstoffatom oder ein Kation; R ist ein Substituent; und n bedeutet eine ganze Zahl im Bereich von 0 bis 5.

5. Verarbeitungszusammensetzung nach Anspruch 1, wobei die Verarbeitungszusammensetzung eine Bleichlösung oder eine Bleichfixierlösung ist.

6. Verarbeitungszusammensetzung nach Anspruch 1, wobei die Verarbeitungszusammensetzung die Metallchelatverbindung in einer Menge im Bereich von 0,005 bis 1 Mol/l enthält.

7. Verarbeitungszusammensetzung nach Anspruch 1, wobei die Verbindung, die durch die Formel (I) dargestellt wird, ein Bleichmittel ist.

8. Verfahren zur Verarbeitung eines photographischen Silberhalogenidmaterials, umfassend das Verartieiten eines bildweise belichteten photographischen Silberhalogenidmaterials mit der Verarbeitungszusammensetzung nach einem der Ansprüche 1 bis 7.

## Revendications

1. Composition de traitement pour un matériau photographique à l'halogénure d'argent contenant au moins un composé chélate métallique qui agit comme un agent oxydant pour un matériau photographique à l'halogénure d'argent, le composé chélate étant représenté par la formule (I) 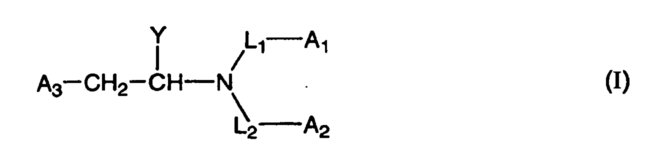 dans laquelle Y représente un groupe hydrocarboné aliphatique cyclique éventuellement substitué, un groupe hétérocyclique insaturé ou saturé de 3 à 10 chaînons ayant au moins un des atomes N, O et S qui peut être monocyclique ou condensé avec un autre cycle pour former un cycle condensé, ou un groupe aryle éventuellement substitué ; L₁ et L₂ représentent chacun un groupe alkylène éventuellement substitué ; et A₁, A₂ et A₃ représentent chacun un groupe carboxyle, un groupe phosphono, un groupe sulfo ou un groupe hydroxy,
à condition que la composition de traitement ne soit pas une solution de traitement ayant une aptitude de blanchiment de l'argent comprenant un sel complexe ferrique de 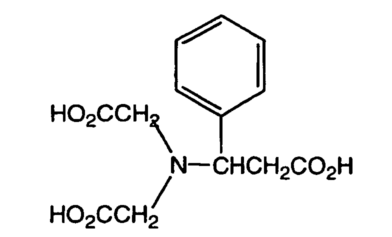 et un persulfate ou un composé de formule 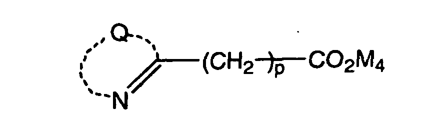 dans laquelle Q représente un groupe atomique non métallique nécessaire pour former un cycle hétérocyclique contenant de l'azote ; p représente 0 ou 1 ; et M₄ représente un atome d'hydrogène ou un cation.

2. Composition de traitement selon la revendication 1, dans laquelle le composé représenté par la formule (I) est représenté par la formule (III) 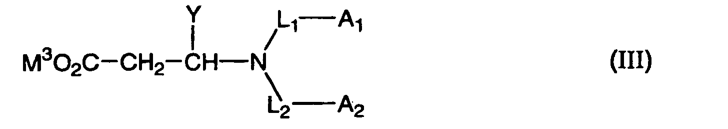 dans laquelle Y, L₁, L₂, A₁ et A₂ ont les mêmes significations que celles dans la formule (I), et M³ représente un atome d'hydrogène ou un cation.

3. Composition de traitement selon la revendication 1, dans laquelle le composé représenté par la formule (I) est représenté par la formule (IV) 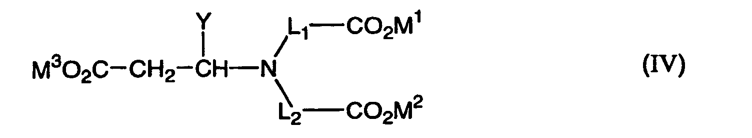 dans laquelle Y, L₁, L₂, A₁ et A₂ ont les mêmes significations que celles dans la formule (I), et M¹, M² et M³ représentent chacun un atome d'hydrogène ou un cation.

4. Composition de traitement selon la revendication 1, dans laquelle le composé représenté par la formule (I) est représenté par la formule (V) 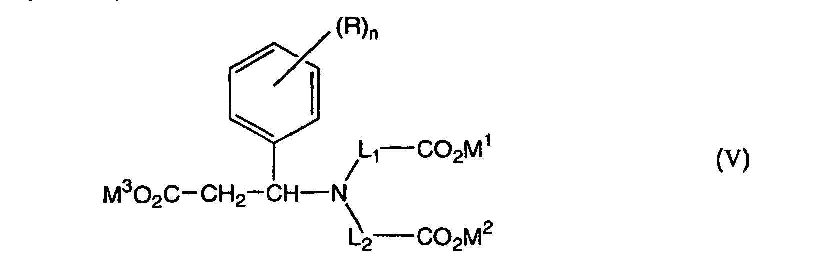 dans laquelle L₁ et L₂ ont les mêmes significations que celles dans la formule (I) ; M¹, M² et M³ représentent chacun un atome d'hydrogène ou un cation ; R représente un substituant ; et n représente un nombre entier de 0 à 5.

5. Composition de traitement selon la revendication 1 qui est une solution de blanchiment ou une solution de blanchiment-fixation.

6. Composition de traitement selon la revendication 1, qui contient le composé chélate métallique en une quantité de 0,005 à 1 mole/l.

7. Composition de traitement selon la revendication 1, dans laquelle le composé de formule (I) est un agent de blanchiment.

8. Procédé de traitement d'un matériau photographique à l'halogénure d'argent comprenant le traitement d'un matériau photographique à l'halogénure d'argent exposé en image avec la composition de traitement selon l'une quelconque des revendications 1 à 7.
